(19) Europäisches Patentamt
European Patent Office
Office européen des brevets

(11) EP 4 338 703 A1

(12) EUROPÄISCHE PATENTANMELDUNG

(43) Veröffentlichungstag:
**20.03.2024 Patentblatt 2024/12**

(21) Anmeldenummer: **23195906.5**

(22) Anmeldetag: **07.09.2023**

(51) Internationale Patentklassifikation (IPC):
***A61C 7/08*** *(2006.01)* ***A61K 6/16*** *(2020.01)*
***A61K 6/887*** *(2020.01)* ***A61K 6/62*** *(2020.01)*
***B33Y 70/00*** *(2020.01)* ***B33Y 80/00*** *(2015.01)*

(52) Gemeinsame Patentklassifikation (CPC):
(C-Sets verfügbar)
**A61K 6/887; A61C 7/08; A61K 6/16; A61K 6/62; B33Y 70/00; B33Y 80/00** (Forts.)

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**
Benannte Erstreckungsstaaten:
**BA**
Benannte Validierungsstaaten:
**KH MA MD TN**

(30) Priorität: **15.09.2022 DE 102022123586**

(71) Anmelder: **VOCO GmbH**
**27472 Cuxhaven (DE)**

(72) Erfinder:
- **Lamping, Sebastian**
  **48153 Münster (DE)**
- **Plaumann, Manfred Thomas**
  **27472 Cuxhaven (DE)**

# (54) OPAZITÄTSUMSCHLAG BEI DRUCKHARZEN

(57) Die vorliegende Erfindung betrifft radikalisch polymerisierbare Zusammensetzungen sowie deren Verwendung in Stereolithographie und/oder 3D-Druck, insbesondere zum 3D-Druck von dentalen Formteilen wie dentalen Modellen, Trays, Try-Ins oder Dentures. Die dentalen, radikalisch polymerisierbaren Zusammensetzungen zeichnen sich dabei durch einen Opazitätsumschlag während der Polymerisation aus.

EP 4 338 703 A1

(52) Gemeinsame Patentklassifikation (CPC): (Forts.)

C-Sets
**A61K 6/887, C08L 33/08;**
**A61K 6/887, C08L 33/26**

## Beschreibung

Die vorliegende Erfindung betrifft eine radikalisch polymerisierbare Zusammensetzung sowie deren Verwendung in Stereolithographie und/oder 3D-Druck, vorzugsweise zum 3D-Druck von dentalen Formteilen wie dentalen Modellen, Trays, Try-Ins oder Dentures.

Die Erfindung wird in den beigefügten Patentansprüchen definiert. Bevorzugte Aspekte der vorliegenden Erfindung ergeben sich überdies aus der nachfolgenden Beschreibung einschließlich der Beispiele.

Soweit für einen erfindungsgemäßen Aspekt (Zusammensetzung oder Verwendung) bestimmte Ausgestaltungen als bevorzugt bezeichnet werden, gelten die entsprechenden Ausführungen jeweils auch für die anderen Aspekte der vorliegenden Erfindung, *mutatis mutandis*. Bevorzugte individuelle Merkmale erfindungsgemäßer Aspekte (wie in den Ansprüchen definiert und/oder in der Beschreibung offenbart) sind miteinander kombinierbar und werden vorzugsweise miteinander kombiniert, sofern sich im Einzelfall für den Fachmann aus dem vorliegenden Text nichts anderes ergibt.

Mit der stetigen Weiterentwicklung der digitalen Zahnmedizin haben sich generative Fertigungsverfahren inzwischen sowohl in Zahnarztpraxen als auch in Dentallabors etabliert. Daten der individuellen Zahnsituation werden oftmals durch 3D-Scanner gewonnen und können am Computer bearbeitet werden. Mittels 3D-Druck oder Stereolithographie können auf Basis dieser Daten dann je nach Anwendung dentale Formteile wie beispielsweise Modelle, Trays, Dentures, Splints, Schienen, orthodontische Formteile, IBTs, Casts, Bohrschablonen, Kronen oder Brücken gedruckt werden.

Generative Fertigungsverfahren, speziell auch stereolithographische Verfahren zur Herstellung dentaler Formkörper, sind aus dem Stand der Technik bekannt.

In der WO 97/29901 A1 wird ein Verfahren zur Herstellung eines dreidimensionalen Gegenstandes aus einem härtbaren flüssigen Medium angegeben, wobei der Gegenstand Schicht für Schicht aufgebaut wird, indem jedes Mal eine Schicht flüssigen Mediums auf einen Träger und/oder einen bereits geformten Teil des Gegenstandes in einem flüssiges Medium enthaltenden Behälter angebracht wird und anschließend besagte Schicht gehärtet wird.

Die WO 2013/153183 A2 beschreibt Kompositharzzusammensetzungen und Verfahren zur Herstellung dentaler Bauteile mittels Stereolithographie. Beansprucht wird die Verwendung einer Dentalzusammensetzung, umfassend ein polyreaktives Bindemittel, zwei Photopolymerisationsinitiatoren mit unterschiedlichen Absorptionsmaxima und einen Absorber.

Für einige Anwendungen müssen die gedruckten dentalen Formteile opak sein. Beispielsweise benötigen dentale Modelle und Trays eine gewisse Opazität, um damit arbeiten zu können. Kronen, Brücken, Try-Ins und Dentures benötigen ebenfalls Opazität, um die natürliche Situation im Mund abzubilden.

Dentalmaterialien, die nach der Aushärtung eine gewisse Opazität aufweisen sind aus dem Stand der Technik bekannt. Insbesondere Füllungsmaterialien müssen, um einen ästhetischen, zahnähnlichen Eindruck zu machen, die natürliche Opazität des Zahns widerspiegeln. Gleichzeitig müssen sie durchlässig für das Polymerisationslicht sein, um möglichst vollständig auszuhärten.

Die WO 2016/026915 A1 beschreibt ein dentales Füllungsmaterial, das vor der Aushärtung im Wesentlichen transparent (für die bei der Polymerisation verwendete Wellenlänge) ist und das nach der Aushärtung opak ist und damit der natürlichen Zahnfarbe entspricht. Gelöst wird dieses Problem durch die Auswahl der Brechungsindices von Monomermatrix und Füllstoffen. Vor der Polymerisation stimmen die Brechungsindices gut miteinander überein und das Material erscheint transparent. Durch die Polymerisation und die dabei auftretende Schrumpfung erhöht sich der Brechungsindex der Monomermatrix beim Übergang zum Polymer. Nach der Polymerisation stimmen die Brechungsindices von Polymermatrix und Füllstoff nicht mehr miteinander überein und das Material erscheint opak.

Dieses Verfahren lässt sich zwar gut bei hochgefüllten dentalen Füllungsmaterialien anwenden, es lässt sich aber nicht auf polymerisierbare Zusammensetzungen für den 3D-Druck übertragen. Um diesen Effekt zu zeigen, benötigen die Füllstoffe eine gewisse Partikelgröße. Solche Füllstoffe sind in den dünnflüssigen 3D-Druck-Zusammensetzungen aber nicht mehr ausreichend sedimentationsstabil. Kleinere nanoskalige Füllstoffe sind zwar sedimentationsstabiler, sie zeigen aber nicht unbedingt die gleichen Effekte. Da die Partikelgröße deutlich unterhalb der Wellenlänge des sichtbaren Lichts liegt, erscheinen solche Materialien auch nach Aushärtung oft transparent. Zum anderen sind solche nanoskaligen Füllstoffe aufwendig herzustellen und dementsprechend teuer.

Es besteht also der Bedarf an einfachen, kostengünstigen Zusammensetzungen, die vor Aushärtung transparent und damit gut durchlässig für das Polymerisationslicht sind und die nach Aushärtung eine ausreichende Opazität für die dentale Anwendung aufweisen.

Gelöst wird die Aufgabe durch eine radikalisch polymerisierbare Zusammensetzung umfassend

A) 10 bis 80 Gew.-% polymerisierbare (Meth)acryl-Verbindungen der Formel I

$$Y\text{-}O\text{-}[(C_3H_6O)_v\text{-}L^1]_n\text{-}(C_3H_6O)_w\text{-}Z \qquad (I)$$

wobei

$Y = H_2C=C(R^1)-C(=O)-$ oder $H_2C=C(R^1)-C(=O)-X-L^2-$ ,
$R^1$ = H oder $CH_3$,
X = O oder NH,
$L^1$ = divalente organische Linkergruppe,
$L^2$ = divalente organische Linkergruppe,
Z = Y, H oder C1 bis C4-Alkyl,
v = 36 bis 100,
w = 36 bis 200,
v+w = 75 bis 200,
n = 0 bis 3,

B) 15 bis 85 Gew.-% polymerisierbare (Meth)acryl-Verbindungen der Formel II

$$\{H_2C=C(R^1)-C(=O)-X-[CH_2CH(R^2)O]_m\}_qQ \qquad (II)$$

wobei

$R^1$ = H oder $CH_3$,
$R^2$ = H oder $CH_3$,
X = O oder NH,
Q = ein q-valenter organischer Rest,
m = 1 bis 20,
q = 1 bis 4, und

C) 0.01 bis 5 Gew.-% eines Initiators oder Initiatorsystems für die radikalische Polymerisation,

jeweils bezogen auf die Gesamtmasse der polymerisierbaren Zusammensetzung.

Unter (Meth)acryl-Verbindungen werden dabei sowohl Acryl-Verbindungen als auch Methacryl-Verbindungen verstanden.

An sich ist der Einsatz von Monomeren mit Polypropylengruppen im 3D-Druck bekannt. So beschreibt die WO 2019/175716 A1 mehrere entsprechende Monomere. Allerdings zeigen die dort genannten Monomere nicht den gewünschten Effekt. Wahrscheinlich sind die PPG-Einheiten nicht groß genug.

Auch die WO 2022/097667 A1 beschreibt lichthärtbare Harzzusammensetzungen mit Polyalkylenglycol-Monomeren. Die Zusammensetzungen werden für die Herstellung von Modellen in Gießverfahren eingesetzt und zeichnen sich durch geringe Rußentwicklung aus, was zur Reduzierung von Sprüngen führen soll. Ein Opazitätsumschlag wird nicht beschrieben. Längerkettige Monomere sind nur in Zusammenhang mit PEG-Einheiten offenbart.

Überraschenderweise hat sich gezeigt, dass die Kombination einer (Meth)acryl-Verbindung der Formel (I), die eine oder mehrere große PPG-Gruppen aufweist, und einer (Meth)acryl-Verbindung der Formel (II), die eine oder mehrere Ethylenoxid- oder Propylenoxid-Gruppen aufweist, zu Zusammensetzungen führt, die vor der Polymerisation transparent und nach der Polymerisation opak sind.

Unter transparent wird dabei verstanden, dass die flüssige Monomermischung für Licht im sichtbaren Wellenlängenbereich im Wesentlichen durchlässig ist. Dies gilt insbesondere für die anwendungsrelevanten Schichtstärken (beispielsweise die Füllhöhe der Harzwanne eines 3D-Druckers). Messbar ist die Transluzenz oder die entsprechende Opazität mittels eines Spektralphotometers wie unten beschrieben. Im Wesentlichen durchlässig bedeutet dabei, dass die Transluzenz unter den angegebenen Messbedingungen ≥90%, bevorzugt ≥95%, besonders bevorzugt ≥97%, und ganz besonders bevorzugt ≥99%, ist. Dementsprechend ist die Opazität unter den angegebenen Messbedingungen <10%, bevorzugt <5%, besonders bevorzugt <3%, und ganz besonders bevorzugt <1%.

Unter opak wird dabei verstanden, dass die festen, polymerisierten Prüfkörper für Licht im sichtbaren Wellenlängenbereich überwiegend undurchlässig sind. Dies gilt insbesondere für die anwendungsrelevanten Schichtstärken (beispielsweise die Schichtstärke einer Krone oder eines Trays). Messbar ist die Opazität mittels eines Spektralphotometers wie unten beschrieben. Überwiegend undurchlässig bedeutet dabei, dass die Opazität unter den angegebenen Messbedingungen >50%, bevorzugt >60%, und besonders bevorzugt >70%, ist.

Ein weiterer Vorteil der erfindungsgemäßen Zusammensetzungen ist, dass polymere Fragmente, wie sie z.B. durch Abriss von der Bauplattform entstehen können, durch ihre Opazität leicht im transparenten Harz identifiziert und entfernt werden können, während transparente Fragmente in transparenten Harzen oder opake Fragmente in opaken

Harzen leicht übersehen werden können.

Polymerisierbare (Meth)acryl-Verbindung (A)

Eine erfindungsgemäße Zusammensetzung enthält 10 bis 80 Gew.-%, bevorzugt 10 bis 70 Gew.-%, besonders bevorzugt 10 bis 60 Gew.-%, ganz besonders bevorzugt 10 bis 50 Gew.-%, polymerisierbare (Meth)acryl-Verbindungen der Formel I

$$Y\text{-}O\text{-}[(C_3H_6O)_v\text{-}L^1]_n\text{-}(C_3H_6O)_w\text{-}Z \qquad (I)$$

wobei

$Y = H_2C{=}C(R^1)\text{-}C({=}O)\text{-}$ oder $H_2C{=}C(R^1)\text{-}C({=}O)\text{-}X\text{-}L^2\text{-}$ ,
$R^1$ = H oder $CH_3$,
X = O oder NH,
$L^1$ = divalente organische Linkergruppe,
$L^2$ = divalente organische Linkergruppe,
Z = Y, H oder C1 bis C4-Alkyl,
v = 36 bis 100,
w = 36 bis 200,
v+w = 75 bis 200 und
n = 0 bis 3.

Dabei sind die Gruppen $(C_3H_6O)_v$ und $(C_3H_6O)_w$ Polypropylenglycolgruppen (PPG-Gruppen).

In einer bevorzugten Ausführungsform ist in der (Meth)acryl-Verbindung der Formel I X = O, Z = Y, n = 0 und w = 75 bis 180, bevorzugt 75 bis 150, besonders bevorzugt 100 bis 150.

In einer weiteren bevorzugten Ausführungsform ist in der (Meth)acryl-Verbindung der Formel I X = O, Z = Y, n = 1 bis 3, v = 36 bis 100, bevorzugt 36 bis 90, besonders bevorzugt 36 bis 80, w = 36 bis 100, bevorzugt 36 bis 90, besonders bevorzugt 36 bis 80 und v+w = 75 bis 180, bevorzugt 75 bis 150, besonders bevorzugt 100 bis 150.

In einer weiteren bevorzugten Ausführungsform ist in der (Meth)acryl-Verbindung der Formel I X = O, Z = Y, n = 0 bis 3, v = 36 bis 100, bevorzugt 36 bis 90, besonders bevorzugt 36 bis 80, w = 36 bis 100, bevorzugt 36 bis 90, besonders bevorzugt 36 bis 80 und n*v+w = 75 bis 200, bevorzugt 75 bis 180, besonders bevorzugt 100 bis 150.

In einer bevorzugten Ausführungsform ist in der (Meth)acryl-Verbindung der Formel I $L^1$ eine geradkettige, verzweigte und/oder cyclische Alkylen-, Arylen-, Arylalkylen- oder Alkylarylengruppe, die -O-, -OC(=O)-, -C(=O)O-, -NHC(=O)-, -C(=O)NH-, -OC(=O)NH-, oder-NHC(=O)O-enthalten kann, und
$L^2$ eine geradkettige, verzweigte und/oder cyclische Alkylen-, Arylen-, Arylalkylen- oder Alkylarylengruppe, die-O-, -OC(=O)-, -C(=O)O-, -NHC(=O)-, -C(=O)NH- , -OC(=O)NH-, oder-NHC(=O)O- enthalten kann,
vorzugsweise ist $L^1$ und/oder $L^2$ ausgewählt aus der Gruppe bestehend aus

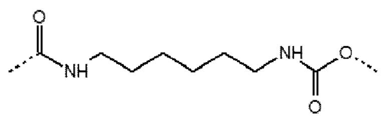 , 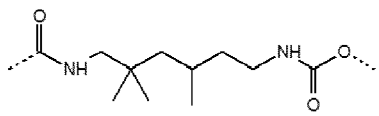 ,

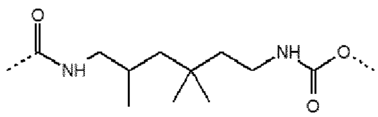 , 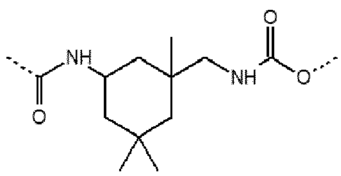 ,

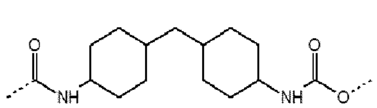 , 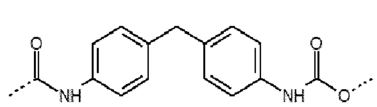 ,

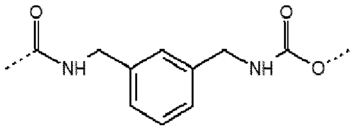 und 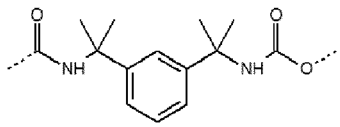 ,

bevorzugt ausgewählt aus der Gruppe bestehend aus

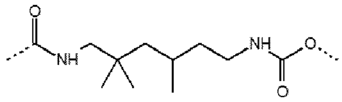 , 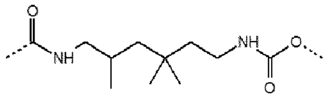 und 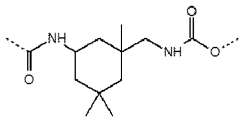 .

Dabei ist $L^1$ mit der linken gestrichelten Linie an das äußere Sauerstoffatom der Polypropylengruppe $(C_3H_6O)_v$ gebunden und mit der rechten gestrichelten Linie an das äußere Kohlenstoffatom der Polypropylengruppe $(C_3H_6O)_w$ gebunden und/oder

$L^2$ ist mit der linken gestrichelten Linie an X gebunden und mit der rechten gestrichelten Linie überein Sauerstoffatom ( -O- ) an das äußere Kohlenstoffatom einer der Polypropylengruppen $(C_3H_6O)_w$ oder $(C_3H_6O)_v$ gebunden.

In einer bevorzugten Ausführungsform weist die (Meth)acryl-Verbindung der Formel I ein Molekulargewicht von größer 5000 g/mol, bevorzugt von größer 6000 g/mol, besonders bevorzugt von größer 7000 g/mol, und/oder im Bereich von 5000 bis 15000 g/mol, bevorzugt im Bereich von 6000 bis 12000 g/mol, besonders bevorzugt im Bereich von 7000 bis 10000 g/mol, auf.

Polymerisierbare (Meth)acryl-Verbindung (B)

Eine erfindungsgemäße Zusammensetzung enthält 15 bis 85 Gew.-%, bevorzugt 20 bis 85 Gew.-%, besonders bevorzugt 30 bis 85 Gew.-%, ganz besonders bevorzugt 40 bis 85 Gew.-%, polymerisierbare (Meth)acryl-Verbindungen der Formel II

$$\{H_2C{=}C(R^1)\text{-}C({=}O)\text{-}X\text{-}[CH_2CH(R^2)O]_m\}_qQ \qquad \text{(II)}$$

wobei

$R^1$ = H oder $CH_3$,
$R^2$ = H oder $CH_3$, bevorzugt H,
X = O oder NH, bevorzugt O,
Q = ein q-valenter organischer Rest,
m = 1 bis 20 und
q = 1 bis 4.

Vorzugsweise ist X = O und/oder $R^2$ = H.

In einer bevorzugten Ausführungsform ist Q = H, C1 bis C4-Alkyl oder Phenyl, wenn q = 1 oder

Q = $-(CH_2CH_2)-$, $-(CH_2CH(CH_3))-$ oder

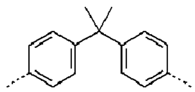 ,

wenn q = 2 oder

Q =

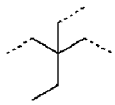

,

wenn q = 3 oder

Q =

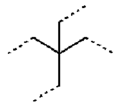

,

wenn q = 4.

Bevorzugte polymerisierbare (Meth)acryl-Verbindungen der Formel (II) mit q = 1 sind 2-Hydroxyethyl(meth)acrylat, 2-Hydroxypropyl(meth)acrylat, 2-(2-Hydroxy-ethoxy)ethyl(meth)acrylat, 2-[2-(2-Hydroxyethoxy)ethoxy]ethyl(meth)acrylat, ethoxyliertes 2-Hydroxyethyl(meth)acrylat, 2-Phenoxyethyl(meth)acrylat, 2-(2-Phenoxyethoxy)ethyl(meth)acrylat, 2-[2-(2-Phenoxyethoxy)ethoxy]ethyl(meth)-acrylat und ethoxyliertes 2-Phenoxyethyl(meth)acrylat.

Bevorzugte polymerisierbare (Meth)acryl-Verbindungen der Formel (II) mit q = 2 sind Ethylenglycoldi(meth)acrylat, Diethylenglycoldi(meth)acrylat, Triethylen-glycoldi(meth)acrylat, Tetraethylenglycoldi(meth)acrylat, Polyethylenglycoldi-(meth)acrylat, Propylenglycoldi(meth)acrylat, Dipropylenglycoldi(meth)acrylat, Tripropylenglycoldi(meth)acrylat, Tetrapropylenglycoldi(meth)acrylat, Poly-propylenglycoldi(meth)acrylat, ethoxyliertes Bisphenol A Di(meth)acrylat und propoxyliertes Bisphenol A Di(meth)acrylat.

Bevorzugte polymerisierbare (Meth)acryl-Verbindungen der Formel (II) mit q = 3 sind ethoxyliertes Trimethylolpropantri(meth)acrylat und propoxyliertes Trimethylolpropantri(meth)acrylat.

Bevorzugte polymerisierbare (Meth)acryl-Verbindungen der Formel (II) mit q = 4 sind ethoxyliertes Pentaerythritoltetra(meth)acrylat und propoxyliertes Pentaerythritoltetra(meth)acrylat.

In einer bevorzugten Ausführungsform machen die Alkylenoxideinheiten $-[CH_2CH(R^2)O]_m-$ mindestens 30 Gew.-% des Molekulargewichts der (Meth)acryl-Verbindung der Formel II aus.

Neben den (Meth)acryl-Verbindungen (I) und (II) kann eine erfindungsgemäße Zusammensetzung auch weitere polymerisierbare Monomere (D), vorzugsweise ebenfalls (Meth)acryl-Verbindungen, enthalten, die nicht den Formeln (I) oder (II) entsprechen. Vorzugsweise liegt der Anteil der polymerisierbaren Monomere (D) im Bereich von 0 bis 50 Gew.-%, bevorzugt im Bereich von 0 bis 30 Gew.-%, besonders bevorzugt im Bereich von 0 bis 10 Gew.-%, jeweils bezogen auf die Gesamtmasse der polymerisierbaren Zusammensetzung. Auf diese Weise ist sichergestellt, dass der synergistische Effekt der (Meth)acryl-Verbindungen (I) und (II) zum Tragen kommt. Vorzugsweise ist der Anteil der polymerisierbaren Monomere (D) kleiner als 50 Gew.-%, bevorzugt kleiner als 30 Gew.-%, besonders bevorzugt kleiner als 10 Gew.-%, jeweils bezogen auf die Gesamtmasse der polymerisierbaren Zusammensetzung.

Bevorzugte Monomere (D) sind, 1,3-Butandioldi(meth)acrylat, 1,4-Butan-dioldi(meth)acrylat, 1,6-Hexandioldi(meth)acrylat, 1,10-Decandioldi(meth)acrylat, 1,12-Dodecandioldi(meth)acrylat, 1,4-Cyclohexandioldi(meth)acrylate, Isobornyl-(meth)acrylat, 2-Hydroxypropyl-1,3-di(meth)acrylat, 3-Hydroxypropyl-1,2-di-(meth)acrylat, Neopentylglycoldi(meth)acrylat, Trimethylolpropantri(meth)acrylat, Pentaerythritoldi(meth)acrylat, Pentaerythritoltri(meth)acrylat, Pentaerythritol-tetra(meth)acrylat, 2,2-Bis[4-[3-(meth)acryloyloxy-2-hydroxypropoxy]phenyl]-propan, 3,14-Dioxa-4,13-dioxo-5,12-diazahexadecan-1, 16-diyldi(meth)acrylat, 7,7,9-Trimethyl-3,14-dioxa-4,13-dioxo-5,12-diazahexadecan-1,16-diyldi(meth)-acrylat, 7,9,9-Trimethyl-3,14-dioxa-4,13-dioxo-5,12-diazahexadecan-1,16-diyldi-(meth)acrylat, 1,5,5-Trimethyl-1-[(2-(meth)acryloyloxyethyl)carbamoylmethyl]-3-(2-(meth)acryloyloxyethyl)carbamoylcyclohexan (CAS 42405-01-6 Methacrylat; CAS 42404-50-2 Acrylat), 1,1'-[Methylenbis(4,1-phenyleniminocarbonyloxy-2,1-ethandiyl)]bis(meth)acrylat (CAS 51243-61-9 Methacrylat; CAS 69790-08-5 Acrylat), 1,1'-[Methylenbis(2,1-phenyleniminocarbonyloxy-2,1-ethandiyl)]bis-(meth)acrylat und 3(4),8(9)-Bis((meth)acryloyloxymethyl)tricyclo[5.2.1.0$^{2,6}$]decan.

In einer weiteren Ausgestaltung kann die polymerisierbare Zusammensetzung als polymerisierbare Monomere auch ausschließlich (Meth)acryl-Verbindungen enthalten, die den Formeln (I) oder (II) entsprechen. Solche Zusammensetzungen sind somit frei von weiteren polymerisierbaren Monomeren (D).

Eine erfindungsgemäße Zusammensetzung enthält 0.01 bis 5 Gew.-%, bevorzugt 0.1 bis 5 Gew.-%, besonders bevorzugt 0.5 bis 4 Gew.-%, jeweils bezogen auf die Gesamtmasse der polymerisierbaren Zusammensetzung, eines Initiators oder Initiatorsystems für die radikalische Polymerisation. Vorzugsweise ist der Initiator (C) ein Photoinitiator. Als Photoinitiatoren können die für (Meth)acryl-Systeme üblichen, geeigneten Verbindungen eingesetzt werden. Vorteilhaft werden alpha-Diketone, Benzoinalkylether, Thioxanthone, Benzophenone, Acetophenone, Acylphosphinoxide oder Acylgermanium-Verbindungen eingesetzt. Bevorzugt werden Monoacylphosphinoxide oder Bisacylphosphinoxide verwendet.

In einer bevorzugten Ausgestaltung ist (C) ausgewählt aus der Gruppe bestehend aus alpha-Diketonen, Benzoinalkylethern, Thioxanthonen, Benzophenonen, Acetophenonen, Acylphosphinoxiden, und Acylgermanium-Verbindungen, bevorzugt ausgewählt aus der Gruppe bestehend aus 1-Hydroxycyclohexylbenzophenon, 4-(2-Hydroxyethoxy)phenyl-(2-hydroxy-2-propyl)keton, 2-Hydroxy-2-methylpropiophenon, 2,4,6-Trimethylbenzoyldiphenylphosphinoxid, Phenyl-bis(2,4,6-trimethylbenzoyl)phosphinoxid, Ethyl phenyl(2,4,6-trimethylbenzoyl)phosphinate (CAS 84434-11-7) und (Poly(oxy-1,2-ethandiyl),α,α',α"-1,2,3-propantriyltris[w-[[phenyl(2,4,6-trimethylbenzoyl)phosphinyl]oxy]-Polymer) (CAS 1834525-17-5), besonders bevorzugt ausgewählt aus der Gruppe bestehend aus 2,4,6-Trimethylbenzoyldiphenylphosphinoxid, Phenyl-bis(2,4,6-trimethylbenzoyl)phosphinoxid, Ethyl phenyl(2,4,6-trimethylbenzoyl)phosphinate und (Poly(oxy-1,2-ethandiyl),α,α',α"-1,2,3-propantriyltris[ω-[[phenyl(2,4,6-trimethylbenzoyl)phosphinyl]oxy]-Polymer).

In einer bevorzugten Ausführungsform enthält eine erfindungsgemäße Zusammensetzung 0.001 bis 1 Gew.-%, bevorzugt 0.01 bis 0.5 Gew.-%, besonders bevorzugt 0.01 bis 0.3 Gew.-%, jeweils bezogen auf die Gesamtmasse der polymerisierbaren Zusammensetzung, eines oder mehrerer Stabilisatoren (E).

Vorzugsweise ist (E) ausgewählt aus der Gruppe bestehend aus Hydrochinon, Hydrochinonmonomethylether, 2,6-Di-tert.-butyl-4-methylphenol, tert.-Butylhydroxyanisol und 2,2,6,6-Tetramethyl-piperidin-1-oxyl.

Um die Farbe des Materials für die gewünschte Anwendung einzustellen kann eine erfindungsgemäße Zusammensetzung zusätzlich ein oder mehrere Farbmittel (F) enthalten. Als Farbmittel können anorganische Farbpigmente sowie organische Farbpigmente oder Farbstoffe eingesetzt werden. Für zahnfarbene Anwendungen werden gelbe, rote und braune Farbmittel eingesetzt. Für Dentures werden rote Farbmittel eingesetzt. Für Modelle werden beige, braune oder graue Farbmittel eingesetzt. Für Trays können auch grüne oder blaue Farbmittel eingesetzt werden.

Vorteil der erfindungsgemäßen Zusammensetzungen ist, dass die Opazität bereits durch die Polymerisation entsteht und so im Wesentlichen auf den Einsatz von Weißpigmenten verzichtet werden kann. Es ist daher nur ein sehr geringer Anteil an Farbmitteln nötig.

In einer bevorzugten Ausführungsform enthält eine erfindungsgemäße Zusammensetzung 0.0001 bis 1 Gew.-%, bevorzugt 0.0001 bis 0.5 Gew.-%, besonders bevorzugt 0.0001 bis 0.1 Gew.-%, jeweils bezogen auf die Gesamtmasse der polymerisierbaren Zusammensetzung, eines oder mehrerer Farbmittel (F).

In einer bevorzugten Ausführungsform umfasst eine erfindungsgemäße Zusammensetzung

A) in einer Menge von 10 bis 70 Gew.-%, bevorzugt 10 bis 60 Gew.-%,
B) in einer Menge von 20 bis 85 Gew.-%, bevorzugt 30 bis 85 Gew.-% und
C) in einer Menge von 0.1 bis 5 Gew.-%, bevorzugt 0.5 bis 4 Gew.-%, jeweils bezogen auf die Gesamtmasse der polymerisierbaren Zusammensetzung.

In einer weiteren bevorzugten Ausführungsform umfasst eine erfindungsgemäße Zusammensetzung

A) in einer Menge von 10 bis 70 Gew.-%, bevorzugt 10 bis 60 Gew.-%,
B) in einer Menge von 20 bis 85 Gew.-%, bevorzugt 30 bis 85 Gew.-%,
C) in einer Menge von 0,1 bis 5 Gew.-%, bevorzugt 0.5 bis 4 Gew.-%,
D) in einer Menge von 0 bis 30 Gew.-%, bevorzugt 0 bis 10 Gew.-% und
E) in einer Menge von 0.01 bis 0.5 Gew.-%, bevorzugt 0.01 bis 0.3 Gew.-%, jeweils bezogen auf die Gesamtmasse der polymerisierbaren Zusammensetzung.

In einer weiteren bevorzugten Ausführungsform umfasst eine erfindungsgemäße Zusammensetzung

A) in einer Menge von 10 bis 70 Gew.-%, bevorzugt 10 bis 60 Gew.-%,
B) in einer Menge von 20 bis 85 Gew.-%, bevorzugt 30 bis 85 Gew.-%,
C) in einer Menge von 0.1 bis 5 Gew.-%, bevorzugt 0.5 bis 4 Gew.-%,
D) in einer Menge von 0 bis 30 Gew.-%, bevorzugt 0 bis 10 Gew.-%,
E) in einer Menge von 0.01 bis 0.5 Gew.-%, bevorzugt 0.01 bis 0.3 Gew.-%, und
F) in einer Menge von 0.0001 bis 0.5 Gew.-%, bevorzugt 0.0001 bis 0.1 Gew.- %,

jeweils bezogen auf die Gesamtmasse der polymerisierbaren Zusammensetzung.

Wie oben beschrieben resultiert aus dem Zusammenspiel der Monomere (A) und (B) ein Druckharz, das bei der Polymerisation einen Umschlag von einer im Wesentlichen transparenten Monomermischung zu einem im Wesentlichen opaken Polymer zeigt, so dass die beschriebenen Vorteile zum Tragen kommen.

In einer bevorzugten Ausführungsform weist eine erfindungsgemäße radikalisch polymerisierbare Zusammensetzung vor der Polymerisation eine Opazität von kleiner 10% und nach der Polymerisation eine Opazität von größer 50% auf.

In einer besonders bevorzugten Ausführungsform weist eine erfindungsgemäße radikalisch polymerisierbare Zusammensetzung vor der Polymerisation eine Opazität von kleiner 5%, bevorzugt kleiner 3%, und nach der Polymerisation eine Opazität von größer 60%, bevorzugt größer 70%, auf.

Bevorzugt ist eine radikalisch polymerisierbare Zusammensetzung, die vor der Polymerisation unter den angegebenen Messbedingungen eine Opazität <10%, bevorzugt <5%, besonders bevorzugt <3%, und ganz besonders bevorzugt <1%, aufweist. Dementsprechend weist eine solche Zusammensetzung eine Transluzenz ≥90%, bevorzugt ≥95%, besonders bevorzugt ≥97%, und ganz besonders bevorzugt ≥99%, auf.

Bevorzugt ist eine radikalisch polymerisierbare Zusammensetzung, die nach der Polymerisation unter den angegebenen Messbedingungen eine Opazität >50%, bevorzugt >60%, und besonders bevorzugt >70%, aufweist.

Vorzugsweise handelt es sich bei einer erfindungsgemäßen radikalisch polymerisierbare Zusammensetzung um eine dentale, radikalisch polymerisierbare Zusammensetzung.

Erfindungsgemäß ist auch eine radikalisch polymerisierbare Zusammensetzung wie oben beschrieben, zur Verwendung in Stereolithographie und/oder 3D-Druck, vorzugsweise zum 3D-Druck von dentalen Formteilen, bevorzugt zum 3D-Druck von dentalen Modellen, Trays, Try-Ins oder Dentures.

Erfindungsgemäß ist auch die Verwendung einer radikalisch polymerisierbaren Zusammensetzung wie oben beschrieben, in Stereolithographie und/oder 3D-Druck, vorzugsweise zum 3D-Druck von dentalen Formteilen, bevorzugt zum 3D-Druck von dentalen Modellen, Trays, Try-Ins oder Dentures.

Beispiele

Die Ziele und Vorteile dieser Offenbarung werden durch die folgenden Beispiele weiter veranschaulicht, aber die spezifischen Materialien und deren Mengen, die in diesen Beispielen genannt werden, sowie andere Bedingungen und Details sollten nicht so ausgelegt werden, dass sie diese Offenbarung unangemessen einschränken.

Die in den Beispielen verwendeten Materialien werden im Folgenden zusammengefasst.

| | |
|---|---|
| PPG | Polypropylengylcol (CAS 25322-69-4) |
| PEG | Polyethylengylcol (CAS 25322-68-3) |
| HMDI | Hexamethylendiisocyanat (CAS 822-06-0) |
| TMDI | 2,2,4-Trimethylhexamethylendiisocyanat / 2,4,4-Trimethylhexamethylendiisocyanat (Isomerengemisch; CAS 32052-51-0) |
| IPDI | Isophorondiisocyanat (CAS 4098-71-9) |
| DBTL | Dibutylzinndilaurat (CAS 77-58-7) |
| BHT | 2,6-Di-tert.-butyl-4-methylphenol (CAS 128-37-0) |
| HEMA | 2-Hydroxyethylmethacrylat (CAS 868-77-9) |
| HEA | 2-Hydroxyethylacrylat (CAS 818-61-1) |
| PheMA | 2-Phenoxyethylmethacrylat (CAS 10595-06-9) |
| PheA-4 | ethoxyliertes 2-Phenoxyethylacrylat (4 EO-Einheiten; CAS 56641-05-5) |
| TEDMA | Triethylenglycoldimethacrylat (CAS 109-16-0) |
| PEG600DMA | Polyethylenglycol-600-Dimethacrylat (CAS 25852-47-5) |
| BisEMA-6 | ethoxyliertes Bisphenol A Dimethacrylat (6 EO-Einheiten; CAS 41637-38-1) |
| BisEMA-10 | ethoxyliertes Bisphenol A Dimethacrylat (10 EO-Einheiten; CAS 41637-38-1) |
| TMPTA-9 | ethoxyliertes Trimethylolpropantriacrylat (9 EO-Einheiten; CAS 28961-43-5) |
| PETA-4 | ethoxyliertes Pentaerythritoltetraacrylat (4 EO-Einheiten; CAS 51728-26-8) |
| IBOMA | Isobornylmethacrylat (CAS 7534-94-3) |
| IBOA | Isobornylacrylat (CAS 5888-33-5) |
| THFMA | Tetrahydrofurfurylmethacrylat (CAS 2455-24-5) |
| ACMO | Acryloylmorpholin (CAS 5117-12-4) |
| TMPTA | Trimethylolpropantriacrylat (CAS 15625-89-5) |
| PETA | Pentaerythritoltetraacrylat (CAS 4986-89-4) |
| HDDMA | 1,6-Hexandioldimethacrylat (CAS 6606-59-3) |
| TCDDMA | 3(4),8(9)-Bis(methacryloyloxymethyl)tricyclo[5.2.1.$0^{2,6}$]decan (CAS 43048-08-4) |

(fortgesetzt)

| | |
|---|---|
| UDMA | 7,7,9-Trimethyl-3,14-dioxa-4,13-dioxo-5,12-diazahexadecan-1,16-diyldi(meth)acrylat / 7,9,9-Trimethyl-3,14-dioxa-4,13-dioxo-5,12-diazahexadecan-1,16-diyldi(meth)acrylat (Isomerengemisch; CAS 72869-86-4) |
| MonoUDA | 2-[[(Butylamino)carbonyl]oxy]ethylacrylat (CAS 63225-53-6) |
| CTFA | (5-Ethyl-1,3-dioxan-5-yl)methylacrylat (CAS 66492-51-1) |
| IDMA | Isodeyclmethacrylat (CAS 29964-84-9) |

Synthese der Polypropylenglycol(meth)acryl-Verbindungen

Beispiel 1a (PPG-5000-Dimethacrylat):

100.00 g PPG-5000 und 4.05 g Triethylamin werden in 400 ml Chloroform gelöst. 4.18 g Methacryloylchlorid werden in 20 ml Chloroform gelöst und bei 0 °C langsam zugetropft. Nach beendeter Zugabe wird noch eine Stunde bei 0 °C gerührt. Danach wird langsam auf Raumtemperatur erwärmt und für weitere 18 Stunden bei Raumtemperatur gerührt. Der gebildete Niederschlag wird abfiltriert und das Filtrat zweimal mit Wasser, dreimal mit 10%iger Salzsäure und zweimal mit gesättigter Kochsalzlösung gewaschen. Die organische Phase wird über Magnesiumsulfat getrocknet. Es werden 50 mg BHT zugegeben und das Lösungsmittel im Vakuum entfernt. Es wird eine schwach gelbe Flüssigkeit erhalten.

Beispiel 1b (PPG-6000-Dimethacrylat):

120.00 g PPG-6000 und 4.05 g Triethylamin werden in 400 ml Chloroform gelöst. 4.18 g Methacryloylchlorid werden in 25 ml Chloroform gelöst und bei 0 °C langsam zugetropft. Nach beendeter Zugabe wird noch eine Stunde bei 0 °C gerührt. Danach wird langsam auf Raumtemperatur erwärmt und für weitere 18 Stunden bei Raumtemperatur gerührt. Der gebildete Niederschlag wird abfiltriert und das Filtrat zweimal mit Wasser, dreimal mit 10%iger Salzsäure und zweimal mit gesättigter Kochsalzlösung gewaschen. Die organische Phase wird über Magnesiumsulfat getrocknet. Es werden 50 mg BHT zugegeben und das Lösungsmittel im Vakuum entfernt. Es wird eine schwach gelbe Flüssigkeit erhalten.

Beispiel 1c (PPG-5000-Urethan-Dimethacrylat):

200.00 g PPG-5000 werden in 400 ml Chloroform gelöst. Anschließend werden 6.21 g 2-Isocyanatoethylmethacrylat, 200 mg Dibutylzinndilaurat und 100 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten. Viskosität (25 °C): 0.5 Pa*s

Beispiel 1d (PPG-6000-Urethan-Dimethacrylat):

240.00 g PPG-6000 werden in 400 ml Chloroform gelöst. Anschließend werden 6.21 g 2-Isocyanatoethylmethacrylat, 200 mg Dibutylzinndilaurat und 100 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten. Viskosität (25 °C): 0.5 Pa*s

Beispiel 1e (PPG-5000-HMDI-HEMA):

100.00 g PPG-5000, 6.73 g Hexamethylendiisocyanat (HMDI) und 5.21 g 2-Hydroxyethylmethacrylat (HEMA) werden in 200 ml Chloroform gelöst. Anschließend werden 100 mg Dibutylzinndilaurat und 50 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten.

Beispiel 1f (PPG-5000-TMDI-HEA):

100.00 g PPG-5000, 8.41 g Trimethylhexamethylendiisocyanat (TMDI) und 4.64 g 2-Hydroxyethylacrylat (HEA) werden in 200 ml Chloroform gelöst. Anschließend werden 100 mg Dibutylzinndilaurat und 50 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten.

Beispiel 1g (PPG-5000-IPDI-HEMA):

100.00 g PPG-5000, 8.89 g Isophorondiisocyanat (IPDI) und 5.21 g 2-Hydroxyethylmethacrylat (HEMA) werden in 200 ml Chloroform gelöst. Anschließend werden 100 mg Dibutylzinndilaurat und 50 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten.

Beispiel 1h (PPG-5000-IPDI-HEA):

100.00 g PPG-5000, 8.89 g Isophorondiisocyanat (IPDI) und 4.64 g 2-Hydroxyethylacrylat (HEA) werden in 200 ml Chloroform gelöst. Anschließend werden 100 mg Dibutylzinndilaurat und 50 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten.

Beispiel 1i (oligo-PPG-5000-IPDI-HEMA):

100.00 g PPG-3000, 11.12 g Isophorondiisocyanat (IPDI) und 4.38 g 2-Hydroxyethylmethacrylat (HEMA) werden in 200 ml Chloroform gelöst. Anschließend werden 100 mg Dibutylzinndilaurat und 50 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten.

Vergleichsbeispiel V1a (PPG-2000-Dimethacrylat):

40.00 g PPG-2000 und 4.05 g Triethylamin werden in 200 ml Chloroform gelöst. 4.18 g Methacryloylchlorid werden in 25 ml Chloroform gelöst und bei 0 °C langsam zugetropft. Nach beendeter Zugabe wird noch eine Stunde bei 0 °C gerührt. Danach wird langsam auf Raumtemperatur erwärmt und für weitere 18 Stunden bei Raumtemperatur gerührt. Der gebildete Niederschlag wird abfiltriert und das Filtrat zweimal mit Wasser, dreimal mit 10%iger Salzsäure und zweimal mit gesättigter Kochsalzlösung gewaschen. Die organische Phase wird über Magnesiumsulfat getrocknet. Es werden 50 mg BHT zugegeben und das Lösungsmittel im Vakuum entfernt. Es wird eine schwach gelbe Flüssigkeit erhalten.

Vergleichsbeispiel V1b (PPG-1000-Urethan-Dimethacrylat):

80.00 g PPG-1000 werden in 200 ml Chloroform gelöst. Anschließend werden 12.42 g 2-Isocyanatoethylmethacrylat, 200 mg Dibutylzinndilaurat und 100 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten. Viskosität (25 °C): 1.3 Pa*s

Vergleichsbeispiel V1c (PPG-4000-Urethan-Dimethacrylat):

160.00 g PPG-4000 werden in 400 ml Chloroform gelöst. Anschließend werden 12.42 g 2-Isocyanatoethylmethacrylat, 200 mg Dibutylzinndilaurat und 100 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten. Viskosität (25 °C): 0.5 Pa*s

Vergleichsbeispiel V1d (PPG-1000-TMDI-HEMA):

40.00 g PPG-1000, 16.82 g Trimethylhexamethylendiisocyanat (TMDI) und 9.29 g 2-Hydroxyethylacrylat (HEA) werden in 200 ml Chloroform gelöst. Anschließend werden 100 mg Dibutylzinndilaurat und 50 mg BHT zugegeben. Es wird unter Rühren für 6 Stunden auf 55 °C erwärmt. Als Reaktionskontrolle wird das Verschwinden der NCO-Bande bei ca. 2270 $cm^{-1}$ im IR-Spektrum beobachtet. Anschließend wird das Lösungsmittel im Vakuum entfernt. Es wird eine schwach gelbe Flüssigkeit erhalten.

Beispiel V1e (PEG-6000-Dimethacrylat):

120.00 g PEG-6000 und 4.05 g Triethylamin werden in 400 ml Chloroform gelöst. 4.18 g Methacryloylchlorid werden in 25 ml Chloroform gelöst und bei 0 °C langsam zugetropft. Nach beendeter Zugabe wird noch eine Stunde bei 0 °C gerührt. Danach wird langsam auf Raumtemperatur erwärmt und für weitere 18 Stunden bei Raumtemperatur gerührt. Der gebildete Niederschlag wird abfiltriert und das Filtrat zweimal mit Wasser, dreimal mit 10%iger Salzsäure und zweimal mit gesättigter Kochsalzlösung gewaschen. Die organische Phase wird über Magnesiumsulfat getrocknet. Es werden 50 mg BHT zugegeben und das Lösungsmittel im Vakuum entfernt. Es wird eine schwach gelbe Flüssigkeit erhalten.

Messmethoden:

Biegefestigkeit (BF): Die Biegefestigkeiten wurden angepasst an ISO 4049:2009 bestimmt. Aus den Druckharzen wurden mittels 3D-Druck (SolFlex350, W2P Engineering GmbH; Wellenlänge 385 nm, Leistung 8.3 $mW/cm^2$, Pixelgröße 50 μm, Schichtstärke 50 μm) Prüfkörper mit der Abmessung 40 mm x 5 mm x 5 mm hergestellt und mit einem Otoflash (VOCO GmbH) mit 2x2000 Blitzen nachbelichtet. Die Bestimmung der Biegefestigkeit erfolgt bei einer Vorschubgeschwindigkeit von 1 mm/min an einer Zwick-Universalprüfmaschine (Zwick GmbH & Co. KG, Ulm).

Opazität (feste, polymerisierte Proben): Für die Bestimmung der Opazität wurden Prüfkörper mit einem Radius von 10 mm und einer Dicke von 2.3 mm aus den Druckharzen gedruckt (SolFlex350, W2P Engineering GmbH; Wellenlänge 385 nm, Leistung 8.3 $mW/cm^2$, Pixelgröße 50 μm, Schichtstärke 50 μm). Die Prüfkörper wurden mit Schleifpapier der Körnung 2500 und 4000 auf 2.0 mm geschliffen. Zur Bestimmung der Opazität wurden die angefertigten Prüfkörper mit einem Zweistrahl-Spektralphotometer ColorFlex EZ 45/0 der Firma Hunterlab untersucht. Die Untersuchung erfolgte unter Normlicht D65 und unter einem 10° Beobachterwinkel im Reflektionsmodus gegen eine schwarze und eine weiße Kachel.

Opazität (flüssige Proben): Die Bestimmung der Opazität der flüssigen Harze wurde ebenfalls mit einem Zweistrahl-Spektralphotometer ColorFlex EZ 45/0 der Firma Hunterlab durchgeführt. Um eine vorzeitige Polymerisation durch das verwendete Licht auszuschließen wurden die flüssigen Harze ohne Photoinitiatoren vermessen. Die Photoinitiatoren wurden erst anschließend zugegeben. Für die Messung wurden die Harze in eine Glasküvette (Bodenstärke 2.0 mm; Füllhöhe 8.0 mm) gefüllt. Die Untersuchung erfolgte ebenfalls unter Normlicht D65 und unter einem 10° Beobachterwinkel im Reflektionsmodus gegen eine schwarze und eine weiße Kachel.

Die L*a*b*-Werte entstammen der Messung gegen die weiße Kachel. Für die Bestimmung der Opazität und Transluzenz wurden die Tristimulus-Werte $Y_{schwarz}$ und $Y_{weiß}$ aus der Messung gegen die schwarze respektive weiße Kachel verwendet. Die Opazität in Prozent berechnet sich gemäß der Formel

$$Opazität\ [\%] = 100 \times Y_{schwarz}/Y_{weiß}.$$

Viskosität: Gemessen wurde standardmäßig an einem Rheometer der Firma Anton Paar des Typs Physica MCR 301 mit einer 50 mm-Messplatte (Platte/Platte), 0.5 mm Spaltabstand und 1 g Substanz. Vor der Messung wird die Platte auf eine Temperatur von 25 °C temperiert. Die Messdauer beträgt 30 s bei einer Scherrate von 10/s.

Molekulargewicht: Das Molekulargewicht wurde mit Hilfe einer GPC (Agilent Technologies 1260 Infinity) bestimmt. Das System besteht aus einer Vorsäule (Länge 50 mm, Durchmesser 8 mm, Partikelgröße 5 μm) und zwei Hauptsäulen (I: Länge 300 mm, Durchmesser 8 mm, Partikelgröße 5 μm, 50 A; II: Länge 300 mm, Durchmesser 8 mm, Partikelgröße 5 μm, 100 A). Als Laufmittel wird THF verwendet. Als Standard wird Polystyrol verwendet.

Ausführungsbeispiele:

Die in den Tabellen 1 bis 17 aufgeführten Monomere wurden jeweils in den angegebenen Mengen eingewogen und mit einem KPG-Rührwerk für 60 Minuten bei Raumtemperatur gerührt, bis eine homogene Lösung entstanden war. Anschließend erfolgte die Messung Opazität für diese flüssigen Lösungen. Danach wurden die Photoinitiatoren zugegeben und es wurde für weitere 60 Minuten mit einem KPG-Rührwerk bei Raumtemperatur gerührt, bis sich die Photoinitiatoren vollständig gelöst hatten.

Tabelle 1:

| Beispiel | | 2a | 2b | 2c | 2d | 2e | 2f |
|---|---|---|---|---|---|---|---|
| (A) | Monomer 1a | 14.25 | 29.25 | 49.25 | 14.25 | 29.25 | 49.25 |
| (B) | HEMA | 84.25 | 69.25 | 49.25 | - | - | - |
| | HEA | - | - | - | 84.25 | 69.25 | 49.25 |
| (C) | TPO | 1.50 | 1.50 | 1.50 | 1.50 | 1.50 | 1.50 |
| Opazität (monomer) | | 0 | 0 | 0 | 0 | 0 | 0 |
| Opazität (polymer) | | 76 | 80 | 77 | 74 | 74 | 64 |

Tabelle 2:

| Beispiel | | 2g | 2h | 2i | 2j | 2k | 2l |
|---|---|---|---|---|---|---|---|
| (A) | Monomer 1a | 14.25 | 29.25 | 49.25 | 14.25 | 29.25 | 49.25 |
| (B) | PheMA | 84.25 | 69.25 | 49.25 | - | - | - |
| | PheA-4 | - | - | - | 84.25 | 69.25 | 49.25 |
| (C) | TPO | 1.50 | 1.50 | 1.50 | 1.50 | 1.50 | 1.50 |
| Opazität (monomer) | | 0 | 0 | 0 | 0 | 0 | 0 |
| Opazität (polymer) | | 81 | 90 | 84 | 73 | 71 | 62 |

Tabelle 3:

| Beispiel | | 2m | 2n | 2o | 2p | 2q | 2r |
|---|---|---|---|---|---|---|---|
| (A) | Monomer 1a | 14.25 | 29.25 | 49.25 | 14.25 | 29.25 | 49.25 |
| (B) | TEDMA | 84.25 | 69.25 | 49.25 | - | - | - |
| | PEG600DMA | - | - | - | 84.25 | 69.25 | 49.25 |
| (C) | TPO | 1.50 | 1.50 | 1.50 | 1.50 | 1.50 | 1.50 |
| Opazität (monomer) | | 0 | 0 | 0 | 0 | 0 | 0 |
| Opazität (polymer) | | 72 | 85 | 84 | 71 | 82 | 81 |

Tabelle 4:

| Beispiel | | 2s | 2t | 2u | 2v | 2w | 2x |
|---|---|---|---|---|---|---|---|
| (A) | Monomer 1a | 14.25 | 29.25 | 49.25 | 14.25 | 29.25 | 49.25 |
| (B) | BisEMA-6 | 84.25 | 69.25 | 49.25 | - | - | - |
| | BisEMA-10 | - | - | - | 84.25 | 69.25 | 49.25 |
| (C) | TPO | 1.50 | 1.50 | 1.50 | 1.50 | 1.50 | 1.50 |
| Opazität (monomer) | | 0 | 0 | 0 | 0 | 0 | 0 |
| Opazität (polymer) | | 78 | 89 | 75 | 80 | 92 | 92 |

Tabelle 5:

| Beispiel | | 3a | 3b | 3c | 3d | 3e | 3f |
|---|---|---|---|---|---|---|---|
| (A) | Monomer 1a | 14.25 | 29.25 | 49.25 | 14.25 | 29.25 | 49.25 |
| (B) | TMPTA-9 | 84.25 | 69.25 | 49.25 | - | - | - |
| | PETA-4 | - | - | - | 84.25 | 69.25 | 49.25 |
| (C) | TPO | 1.50 | 1.50 | 1.50 | 1.50 | 1.50 | 1.50 |
| Opazität (monomer) | | 0 | 0 | 0 | 0 | 0 | 0 |
| Opazität (polymer) | | 72 | 77 | 81 | 63 | 76 | 81 |

Tabelle 6:

| Beispiel | | 3g | 3h | 3i | 3j | 3k | 3l |
|---|---|---|---|---|---|---|---|
| (A) | Monomer 1a | 14.50 | 24.50 | 34.50 | 14.50 | 24.50 | 34.50 |
| (B) | HEMA | 37.25 | 32.25 | 27.25 | 32.25 | 27.25 | 22.25 |
| | TEDMA | 37.25 | 32.25 | 27.25 | 32.25 | 27.25 | 22.25 |
| (D) | IBOMA | 9.50 | 9.50 | 9.50 | 19.50 | 19.50 | 19.50 |
| (C) | TPO | 1.50 | 1.50 | 1.50 | 1.50 | 1.50 | 1.50 |
| Opazität (monomer) | | 0 | 0 | 0 | 0 | 0 | 0 |
| Opazität (polymer) | | 63 | 67 | 66 | 54 | 59 | 58 |

Tabelle 7:

| Beispiel | | 3m | 3n | 3o | 3p | 3q | 3r |
|---|---|---|---|---|---|---|---|
| (A) | Monomer 1b | 29.25 | - | - | - | - | - |
| | Monomer 1c | - | 29.25 | - | - | - | - |
| | Monomer 1d | - | - | 29.25 | - | - | - |
| | Monomer 1e | - | - | - | 29.25 | - | - |
| | Monomer 1f | - | - | - | - | 29.25 | - |
| | Monomer 1g | - | - | - | - | - | 29.25 |
| (B) | HEMA | 69.25 | 69.25 | 69.25 | 69.25 | 69.25 | 69.25 |
| (C) | TPO | 1.50 | 1.50 | 1.50 | 1.50 | 1.50 | 1.50 |
| Opazität (monomer) | | 0 | 0 | 0 | 0 | | 0 |
| Opazität (polymer) | | 83 | 81 | 83 | 82 | 79 | 84 |

Tabelle 8:

| Beispiel | | 3s | 3t |
|---|---|---|---|
| (A) | Monomer 1h | 29.25 | - |
| | Monomer 1i | - | 29.25 |
| (B) | HEMA | 69.25 | 69.25 |
| (C) | TPO | 1.50 | 1.50 |

(fortgesetzt)

| Beispiel | 3s | 3t |
|---|---|---|
| Opazität (monomer) | 0 | 0 |
| Opazität (polymer) | 83 | 85 |

Tabelle 9:

| Vergleichsbeispiel | | 4a | 4b | 4c | 4d | 4e | 4f |
|---|---|---|---|---|---|---|---|
| (A) | Monomer 1a | 4.25 | 84.25 | 4.25 | 84.25 | 4.25 | 84.25 |
| (B) | HEMA | 94.25 | 14.25 | - | - | - | - |
| | HEA | - | - | 94.25 | 14.25 | - | - |
| | TEDMA | - | - | - | - | 94.25 | 14.25 |
| (C) | TPO | 1.50 | 1.50 | 1.50 | 1.50 | 1.50 | 1.50 |
| Opazität (monomer) | | 0 | 0 | 0 | 0 | 0 | 0 |
| Opazität (polymer) | | 23 | 37 | 18 | 31 | 25 | 38 |

Tabelle 10:

| Vergleichsbeispiel | | 4g | 4h | 4i | 4j | 4k | 4l |
|---|---|---|---|---|---|---|---|
| (A) | Monomer 1a | 14.25 | 29.25 | 49.25 | 14.25 | 29.25 | 49.25 |
| (D) | IBOMA | 84.25 | 69.25 | 49.25 | - | - | - |
| | IBOA | - | - | - | 84.25 | 69.25 | 49.25 |
| (C) | TPO | 1.50 | 1.50 | 1.50 | 1.50 | 1.50 | 1.50 |
| Opazität (monomer) | | 0 | 0 | 0 | 0 | 0 | 0 |
| Opazität (polymer) | | 6 | 12 | 4 | 1 | 1 | 1 |

Tabelle 11:

| Vergleichsbeispiel | | 4m | 4n | 4o | 4p | 4q | 4r |
|---|---|---|---|---|---|---|---|
| (A) | Monomer 1a | 14.25 | 29.25 | 49.25 | 14.25 | 29.25 | 49.25 |
| (D) | THFMA | 84.25 | 69.25 | 49.25 | - | - | - |
| | ACMO | - | - | - | 84.25 | 69.25 | 49.25 |
| (C) | TPO | 1.50 | 1.50 | 1.50 | 1.50 | 1.50 | 1.50 |
| Opazität (monomer) | | 0 | 0 | 0 | 0 | 0 | 0 |
| Opazität (polymer) | | * | 13 | 1 | 2 | 7 | 12 |
| * keine Aushärtung | | | | | | | |

Tabelle 12:

| Vergleichsbeispiel | | 4s | 4t | 4u | 4v | 4w | 4x |
|---|---|---|---|---|---|---|---|
| (A) | Monomer 1a | 14.25 | 29.25 | 49.25 | 14.25 | 29.25 | 49.25 |

(fortgesetzt)

| Vergleichsbeispiel | | 4s | 4t | 4u | 4v | 4w | 4x |
|---|---|---|---|---|---|---|---|
| (D) | TMPTA | 84.25 | 69.25 | 49.25 | - | - | - |
| | PETA | - | - | - | 84.25 | 69.25 | 49.25 |
| (C) | TPO | 1.50 | 1.50 | 1.50 | 1.50 | 1.50 | 1.50 |
| Opazität (monomer) | | 0 | 0 | 0 | 0 | 0 | 0 |
| Opazität (polymer) | | 2 | 7 | 12 | 13 | 17 | 22 |

Tabelle 13:

| Vergleichsbeispiel | | 5a | 5b | 5c | 5d | 5e | 5f |
|---|---|---|---|---|---|---|---|
| (A) | Monomer 1a | 14.25 | 29.25 | 49.25 | 14.25 | 29.25 | 49.25 |
| (D) | HDDMA | 84.25 | 69.25 | 49.25 | - | - | - |
| | TCDDMA | - | - | - | 84.25 | 69.25 | 49.25 |
| (C) | TPO | 1.50 | 1.50 | 1.50 | 1.50 | 1.50 | 1.50 |
| Opazität (monomer) | | 0 | 0 | 0 | 0 | 0 | 0 |
| Opazität (polymer) | | 4 | 30 | 25 | 1 | 3 | 7 |

Tabelle 14:

| Vergleichsbeispiel | | 5g | 5h | 5i | 5j | 5k | 5l |
|---|---|---|---|---|---|---|---|
| (A) | Monomer 1a | 14.25 | 29.25 | 49.25 | 14.25 | 29.25 | 49.25 |
| (D) | UDMA | 84.25 | 69.25 | 49.25 | - | - | - |
| | MonoUDA | - | - | - | 84.25 | 69.25 | 49.25 |
| (C) | TPO | 1.50 | 1.50 | 1.50 | 1.50 | 1.50 | 1.50 |
| Opazität (monomer) | | 0 | 0 | 0 | 0 | 0 | 0 |
| Opazität (polymer) | | 1 | 1 | 24 | 2 | 2 | 3 |

Tabelle 15:

| Vergleichsbeispiel | | 5m | 5n | 5o | 5p | 5q | 5r |
|---|---|---|---|---|---|---|---|
| (A) | Monomer 1a | 14.25 | 29.25 | 49.25 | 14.25 | 29.25 | 49.25 |
| (D) | CTFA | 84.25 | 69.25 | 49.25 | - | - | - |
| | IDMA | - | - | - | 84.25 | 69.25 | 49.25 |
| (C) | TPO | 1.50 | 1.50 | 1.50 | 1.50 | 1.50 | 1.50 |
| Opazität (monomer) | | 0 | 0 | 0 | 0 | 0 | 0 |
| Opazität (polymer) | | 2 | 2 | 2 | * | * | 5 |
| * keine Aushärtung | | | | | | | |

Tabelle 16:

| Vergleichsbeispiel | | 5s | 5t | 5u | 5v | 5w | 5x |
|---|---|---|---|---|---|---|---|
| (A) | Monomer 1a | 9.50 | 9.50 | 9.50 | 9.50 | 9.50 | 9.50 |
| (B) | HEMA | 79.50 | 69.50 | 39.75 | 34.75 | - | - |
| | TEDMA | - | - | 39.75 | 34.75 | 79.50 | 69.50 |
| (D) | IBOMA | 9.50 | 19.50 | 9.50 | 19.50 | 9.50 | 19.50 |
| (C) | TPO | 1.50 | 1.50 | 1.50 | 1.50 | 1.50 | 1.50 |
| Opazität (monomer) | | 0 | 0 | 0 | 0 | 0 | 0 |
| Opazität (polymer) | | 47 | 36 | 48 | 36 | 45 | 34 |

Tabelle 17:

| Vergleichsbeispiel | | 6a | 6b | 6c | 6d | 6e |
|---|---|---|---|---|---|---|
| | Monomer V1a | 29.25 | - | - | - | - |
| | Monomer V1b | - | 29.25 | - | - | - |
| | Monomer V1c | - | - | 29.25 | - | - |
| | Monomer V1d | - | - | - | 29.25 | - |
| | Monomer V1e | - | - | - | - | 29.25 |
| (B) | HEMA | 69.25 | 69.25 | 69.25 | 69.25 | 69.25 |
| (C) | TPO | 1.50 | 1.50 | 1.50 | 1.50 | 1.50 |
| Opazität (monomer) | | 0 | 0 | 0 | 0 | 0 |
| Opazität (polymer) | | 27 | 18 | 43 | 19 | 34 |

Insgesamt lässt sich die Erfindung anhand der folgenden Aspekte zusammenfassen.

1. Radikalisch polymerisierbare Zusammensetzung umfassend

A) 10 bis 80 Gew.-% polymerisierbare (Meth)acryl-Verbindungen der Formel I

$$Y\text{-}O\text{-}[(C_3H_6O)_v\text{-}L^1]_n\text{-}(C_3H_6O)_w\text{-}Z \qquad (I)$$

wobei

$Y = H_2C{=}C(R^1)\text{-}C({=}O)\text{-}$ oder $H_2C{=}C(R^1)\text{-}C({=}O)\text{-}X\text{-}L^2\text{-}$ ,
$R^1$ = H oder $CH_3$,
X = O oder NH,
$L^1$ = divalente organische Linkergruppe,
$L^2$ = divalente organische Linkergruppe,
Z = Y, H oder C1 bis C4-Alkyl,
v = 36 bis 100,
w = 36 bis 200,
v+w = 75 bis 200,
n = 0 bis 3,

B) 15 bis 85 Gew.-% polymerisierbare (Meth)acryl-Verbindungen der Formel II

$$\{H_2C{=}C(R^1)\text{-}C({=}O)\text{-}X\text{-}[CH_2CH(R^2)O]_m\}_qQ \qquad (II)$$

wobei

$R^1$ = H oder $CH_3$,
$R^2$ = H oder $CH_3$,
X = O oder NH,
Q = ein q-valenter organischer Rest,
m = 1 bis 20,
q = 1 bis 4, und

C) 0.01 bis 5 Gew.-% eines Initiators oder Initiatorsystems für die radikalische Polymerisation,

jeweils bezogen auf die Gesamtmasse der polymerisierbaren Zusammensetzung.

2. Radikalisch polymerisierbare Zusammensetzung nach Aspekt 1, umfassend (A) in einer Menge von 10 bis 70 Gew.-%, bevorzugt 10 bis 60 Gew.-%, besonders bevorzugt 10 bis 50 Gew.-%, bezogen auf die Gesamtmasse der polymerisierbaren Zusammensetzung.

3. Radikalisch polymerisierbare Zusammensetzung nach einem der vorangehenden Aspekte, wobei in der (Meth)acryl-Verbindung der Formel I X = O, Z = Y, n = 0 und w = 75 bis 180, bevorzugt 75 bis 150, besonders bevorzugt 100 bis 150 und ganz besonders bevorzugt 103 bis 121.

4. Radikalisch polymerisierbare Zusammensetzung nach einem der vorangehenden Aspekte, wobei in der (Meth)acryl-Verbindung der Formel I X = O, Z = Y, n = 1 bis 3, v = 36 bis 100, bevorzugt 36 bis 90, besonders bevorzugt 36 bis 80, w = 36 bis 100, bevorzugt 36 bis 90, besonders bevorzugt 36 bis 80 und v+w = 75 bis 180, bevorzugt 75 bis 150, besonders bevorzugt 100 bis 150.

5. Radikalisch polymerisierbare Zusammensetzung nach einem der vorangehenden Aspekte, wobei in der (Meth)acryl-Verbindung der Formel I X = O, Z = Y, n = 0 bis 3, v = 36 bis 100, bevorzugt 36 bis 90, besonders bevorzugt 36 bis 80, w = 36 bis 100, bevorzugt 36 bis 90, besonders bevorzugt 36 bis 80 und n*v+w = 75 bis 200, bevorzugt 75 bis 180, besonders bevorzugt 100 bis 150.

6. Radikalisch polymerisierbare Zusammensetzung nach einem der vorangehenden Aspekte, wobei in der (Meth)acryl-Verbindung der Formel I $L^1$ eine geradkettige, verzweigte und/oder cyclische Alkylen-, Arylen-, Arylalkylen- oder Alkylarylengruppe, die -O-, -OC(=O)-, -C(=O)O-, - NHC(=O)-, -C(=O)NH-, -OC(=O)NH-, oder -NHC(=O)O- enthalten kann ist, und
$L^2$ eine geradkettige, verzweigte und/oder cyclische Alkylen-, Arylen-, Arylalkylen- oder Alkylarylengruppe, die -O-, -OC(=O)-, -C(=O)O-, - NHC(=O)-, -C(=O)NH-, -OC(=O)NH-, oder -NHC(=O)O- enthalten kann ist.

7. Radikalisch polymerisierbare Zusammensetzung nach einem der vorangehenden Aspekte, wobei $L^1$ und/oder $L^2$ ausgewählt sind aus der Gruppe bestehend aus

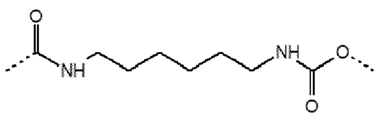 , 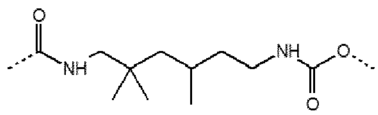 ,

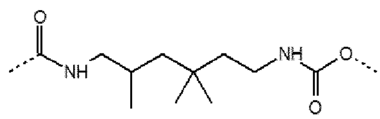 , 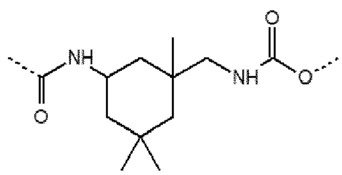 ,

, ,

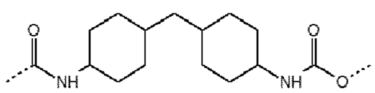 , 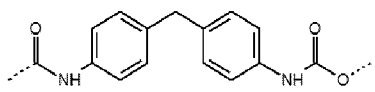 ,

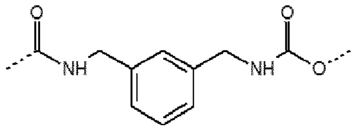 und 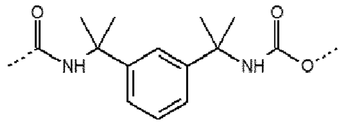 ,

bevorzugt ausgewählt aus der Gruppe bestehend aus

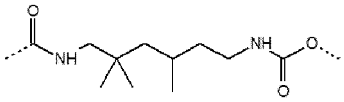 , 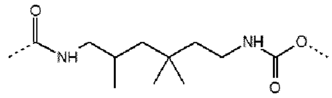

und

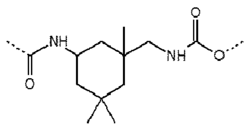 .

8. Radikalisch polymerisierbare Zusammensetzung nach einem der vorangehenden Aspekte, wobei $L^1$ und/oder $L^2$ ausgewählt sind aus der Gruppe bestehend aus

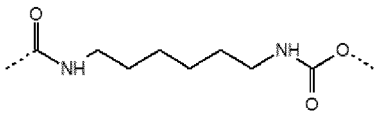 , 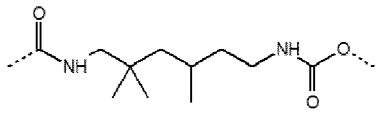 ,

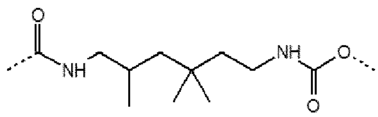 , 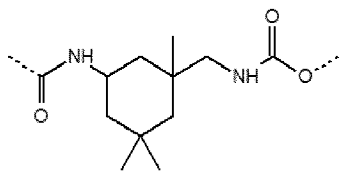 ,

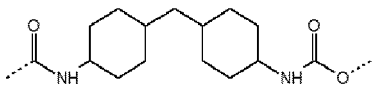 , 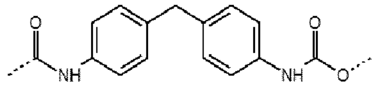 ,

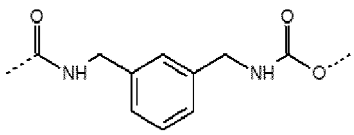 und 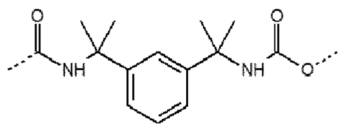 ,

bevorzugt ausgewählt aus der Gruppe bestehend aus

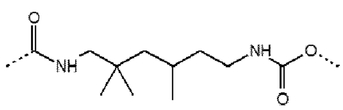 , 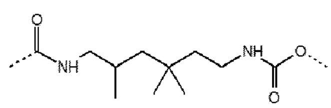

und

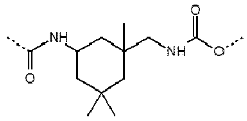

,

wobei $L^1$ mit der linken gestrichelten Linie an das äußere Sauerstoffatom der Polypropylengruppe $(C_3H_6O)_v$ gebunden ist und mit der rechten gestrichelten Linie an das äußere Kohlenstoffatom der Polypropylengruppe $(C_3H_6O)_w$ gebunden ist und/oder
$L^2$ mit der linken gestrichelten Linie an X gebunden ist und mit der rechten gestrichelten Linie über ein Sauerstoffatom ( -O- ) an das äußere Kohlenstoffatom einer der Polypropylengruppen $(C_3H_6O)_w$ oder $(C_3H_6O)_v$ gebunden ist.

9. Radikalisch polymerisierbare Zusammensetzung nach einem der vorangehenden Aspekte, wobei die (Meth)acryl-Verbindung der Formel I ein Molekulargewicht von größer 5000 g/mol, bevorzugt von größer 6000 g/mol, besonders bevorzugt von größer 7000 g/mol, und/oder im Bereich von 5000 bis 15000 g/mol, bevorzugt im Bereich von 6000 bis 12000 g/mol, besonders bevorzugt im Bereich von 7000 bis 10000 g/mol, aufweist.

10. Radikalisch polymerisierbare Zusammensetzung nach einem der vorangehenden Aspekte, wobei die Gruppen $(C_3H_6O)_v$ und $(C_3H_6O)_w$ Polypropylenglycolgruppen, vorzugsweise 1,2-Polypropylenglycolgruppen, sind.

11. Radikalisch polymerisierbare Zusammensetzung nach einem der vorangehenden Aspekte, umfassend (B) in einer Menge von 20 bis 85 Gew.-%, bevorzugt 30 bis 85 Gew.-%, besonders bevorzugt 40 bis 85 Gew.-%, bezogen auf die Gesamtmasse der polymerisierbaren Zusammensetzung.

12. Radikalisch polymerisierbare Zusammensetzung nach einem der vorangehenden Aspekte, wobei in der (Meth)acryl-Verbindung der Formel II X = O und/oder $R^2$ = H.

13. Radikalisch polymerisierbare Zusammensetzung nach einem der vorangehenden Aspekte, wobei in der (Meth)acryl-Verbindung der Formel II Q = H, C1 bis C4-Alkyl oder Phenyl, wenn q = 1 oder

Q = $-(CH_2CH_2)-$, $-(CH_2CH(CH_3))-$,

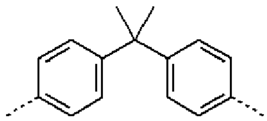

,

wenn q = 2 oder

Q =

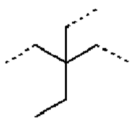

,

wenn q = 3 oder

Q =

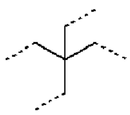

,

wenn q = 4.

14. Radikalisch polymerisierbare Zusammensetzung nach einem der vorangehenden Aspekte, wobei die (Meth)acryl-Verbindungen der Formel (II) mit q = 1 ausgewählt ist aus der Gruppe bestehend aus 2-Hydroxyethyl(meth)acrylat, 2-Hydroxypropyl(meth)acrylat, 2-(2-Hydroxy-ethoxy)ethyl(meth)acrylat, 2-[2-(2-Hydroxyethoxy)ethoxy]ethyl(meth)acrylat, ethoxyliertes 2-Hydroxyethyl(meth)acrylat, 2-Phenoxyethyl(meth)acrylat, 2-(2-Phenoxyethoxy)ethyl(meth)acrylat, 2-[2-(2-Phenoxyethoxy)ethoxy]-ethyl(meth)acrylat und ethoxyliertes 2-Phenoxyethyl(meth)acrylat.

15. Radikalisch polymerisierbare Zusammensetzung nach einem der vorangehenden Aspekte, wobei die (Meth)acryl-Verbindungen der Formel (II) mit q = 2 ausgewählt ist aus der Gruppe bestehend aus Ethylenglycoldi(meth)acrylat, Diethylenglycoldi(meth)acrylat, Triethylen-glycoldi(meth)acrylat, Tetraethylenglycoldi(meth)acrylat, Polyethylenglycoldi-(meth)acrylat, Propylenglycoldi(meth)acrylat, Dipropylenglycoldi(meth)acrylat, Tripropylenglycoldi(meth)acrylat, Tetrapropylenglycoldi(meth)acrylat, Polypropylenglycoldi(meth)acrylat, ethoxyliertes Bisphenol A Di(meth)acrylat und propoxyliertes Bisphenol A Di(meth)acrylat.

16. Radikalisch polymerisierbare Zusammensetzung nach einem der vorangehenden Aspekte, wobei die (Meth)acryl-Verbindungen der Formel (II) mit q = 3 ausgewählt ist aus der Gruppe bestehend aus ethoxyliertem Trimethylolpropantri(meth)acrylat und propoxyliertem Trimethylolpropantri(meth)acrylat.

17. Radikalisch polymerisierbare Zusammensetzung nach einem der vorangehenden Aspekte, wobei die (Meth)acryl-Verbindungen der Formel (II) mit q = 4 ausgewählt ist aus der Gruppe bestehend aus ethoxyliertem Pentaerythritoltetra(meth)acrylat und propoxyliertem Pentaerythritoltetra(meth)acrylat.

18. Radikalisch polymerisierbare Zusammensetzung nach einem der vorangehenden Aspekte, wobei die Alkylenoxideinheiten $-[CH_2CH(R^2)O]_m-$ mit $R^2$ = H oder Methyl mindestens 30 Gew.-% des Molekulargewichts der (Meth)acryl-Verbindung der Formel II ausmachen.

19. Radikalisch polymerisierbare Zusammensetzung nach einem der vorangehenden Aspekte, umfassend (Meth)acryl-Verbindungen (D), die nicht den Formeln (I) oder (II) entsprechen.

20. Radikalisch polymerisierbare Zusammensetzung nach einem der vorangehenden Aspekte, umfassend (Meth)acryl-Verbindungen (D), die nicht den Formeln (I) oder (II) entsprechen, in einer Menge im Bereich von 0 bis 50 Gew.-%, bevorzugt im Bereich von 0 bis 30 Gew.-%, besonders bevorzugt im Bereich von 0 bis 10 Gew.-%, jeweils bezogen auf die Gesamtmasse der polymerisierbaren Zusammensetzung.

21. Radikalisch polymerisierbare Zusammensetzung nach einem der vorangehenden Aspekte, umfassend (Meth)acryl-Verbindungen (D), die nicht den Formeln (I) oder (II) entsprechen, in einer Menge von kleiner 50 Gew.-%, bevorzugt kleiner 30 Gew.-%, besonders bevorzugt von kleiner 10 Gew.-%, jeweils bezogen auf die Gesamtmasse der polymerisierbaren Zusammensetzung.

22. Radikalisch polymerisierbare Zusammensetzung nach einem der vorangehenden Aspekte, wobei (D) ausgewählt ist aus der Gruppe bestehend aus 1,3-Butandioldi(meth)acrylat, 1,4-Butandioldi(meth)acrylat, 1,6-Hexandioldi(meth)acrylat, 1,10-Decandioldi(meth)acrylat, 1,12-Dodecandioldi(meth)acrylat, 1,4-Cyclohexandioldi(meth)acrylate, Isobornyl-(meth)acrylat, 2-Hydroxypropyl-1,3-di(meth)acrylat, 3-Hydroxypropyl-1,2-di(meth)acrylat, Neopentylglycoldi(meth)acrylat, Trimethylolpropantri(meth)-acrylat, Pentaerythritoldi(meth)acrylat, Pentaerythritoltri(meth)acrylat, Penta-erythritoltetra(meth)acrylat, 2,2-Bis[4-[3-(meth)acryloyloxy-2-hydroxypropoxy]phenyl]propan, 3,14-Dioxa-4,13-dioxo-5,12-diazahexadecan-1,16-diyldi(meth)acrylat, 7,7,9-Trimethyl-3,14-dioxa-4,13-dioxo-5,12-diazahexadecan-1,16-diyldi(meth)acrylat, 7,9,9-Trimethyl-3,14-dioxa-4,13-dioxo-5,12-diazahexadecan-1,16-diyldi(meth)acrylat, 1,5,5-Trimethyl-1-[(2-(meth)acryloyloxyethyl)carbamoylmethyl]-3-(2-(meth)acryloyloxyethyl)carbamoylcyclohexan (CAS 42405-01-6 Methacrylat; CAS 42404-50-2 Acrylat), 1,1'-[Methylenbis(4,1-phenyleniminocarbonyloxy-2,1-ethandiyl)]bis(meth)acrylat (CAS 51243-61-9 Methacrylat; CAS 69790-08-5 Acrylat), 1,1'-[Methylenbis(2,1-phenyleniminocarbonyloxy-2,1-ethandiyl)]bis(meth)acrylat und 3(4),8(9)-Bis((meth)acryloyloxymethyl)-tricyclo[5.2.1.$0^{2,6}$]decan.

23. Radikalisch polymerisierbare Zusammensetzung nach einem der vorangehenden Aspekte, umfassend keine (Meth)acryl-Verbindungen (D), die nicht den Formeln (I) oder (II) entsprechen.

24. Radikalisch polymerisierbare Zusammensetzung nach einem der vorangehenden Aspekte, umfassend einen Initiator oder Initiatorsystem (C) in einer Menge von 0.1 bis 5 Gew.-%, bevorzugt 0.5 bis 4 Gew.-%, jeweils bezogen auf die Gesamtmasse der polymerisierbaren Zusammensetzung.

25. Radikalisch polymerisierbare Zusammensetzung nach einem der vorangehenden Aspekte, wobei der Initiator oder das Initiatorsystem (C) einen Photoinitiator umfasst oder aus einem Photoinitiator besteht.

26. Radikalisch polymerisierbare Zusammensetzung nach einem der vorangehenden Aspekte, wobei (C) ausgewählt ist aus der Gruppe bestehend aus alpha-Diketonen, Benzoinalkylethern, Thioxanthonen, Benzophenonen, Acetophenonen, Acylphosphinoxiden, und Acylgermanium-Verbindungen.

27. Radikalisch polymerisierbare Zusammensetzung nach einem der vorangehenden Aspekte, wobei (C) ausgewählt ist aus der Gruppe bestehend aus 1-Hydroxycyclohexylbenzophenon, 4-(2-Hydroxyethoxy)phenyl-(2-hydroxy-2-propyl)keton, 2-Hydroxy-2-methylpropiophenon, 2,4,6-Trimethylbenzoyldiphenylphosphinoxid, Phenyl-bis(2,4,6-trimethylbenzoyl)phosphinoxid, Ethyl phenyl(2,4,6-trimethylbenzoyl)phosphinate (CAS 84434-11-7) und (Poly(oxy-1,2-ethandiyl),α,α',α"-1,2,3-propantriyltris[ω-[[phenyl(2,4,6-trimethylbenzoyl)phosphinyl]oxy]-Polymer) (CAS 1834525-17-5), besonders bevorzugt ausgewählt aus der Gruppe bestehend aus 2,4,6-Trimethylbenzoyldiphenylphosphinoxid, Phenyl-bis(2,4,6-trimethylbenzoyl)phosphinoxid, Ethyl phenyl(2,4,6-trimethylbenzoyl)phosphinate und (Poly(oxy-1,2-ethandiyl),α,α',α"-1,2,3-propantriyltris[w-[[phenyl(2,4,6-trimethylbenzoyl)phosphinyl]oxy]-Polymer).

28. Radikalisch polymerisierbare Zusammensetzung nach einem der vorangehenden Aspekte, umfassend einen oder mehrere Stabilisatoren (E) in einer Menge von 0.001 bis 1 Gew.-%, bevorzugt 0.01 bis 0.5 Gew.-%, besonders bevorzugt 0.01 bis 0.3 Gew.-%, jeweils bezogen auf die Gesamtmasse der polymerisierbaren Zusammensetzung.

29. Radikalisch polymerisierbare Zusammensetzung nach einem der vorangehenden Aspekte, wobei die Stabilisatoren (E) ausgewählt sind aus der Gruppe bestehend aus Hydrochinon, Hydrochinonmonomethylether, 2,6-Ditert.-butyl-4-methylphenol, tert.-Butylhydroxyanisol und 2,2,6,6-Tetramethylpiperidin-1-oxyl.

30. Radikalisch polymerisierbare Zusammensetzung nach einem der vorangehenden Aspekte, umfassend ein oder mehrere Farbmittel (F) in einer Menge von 0.0001 bis 1 Gew.-%, bevorzugt 0.0001 bis 0.5 Gew.-%, besonders bevorzugt 0.0001 bis 0.1 Gew.-%, jeweils bezogen auf die Gesamtmasse der polymerisierbaren Zusammensetzung.

31. Radikalisch polymerisierbare Zusammensetzung nach einem der vorangehenden Aspekte, umfassend

A) in einer Menge von 10 bis 70 Gew.-%, bevorzugt 10 bis 60 Gew.-%,
B) in einer Menge von 20 bis 85 Gew.-%, bevorzugt 30 bis 85 Gew.-% und
C) in einer Menge von 0.1 bis 5 Gew.-%, bevorzugt 0.5 bis 4 Gew.-%, jeweils bezogen auf die Gesamtmasse der polymerisierbaren Zusammensetzung.

32. Radikalisch polymerisierbare Zusammensetzung nach einem der vorangehenden Aspekte, umfassend

A) in einer Menge von 10 bis 70 Gew.-%, bevorzugt 10 bis 60 Gew.-%,
B) in einer Menge von 20 bis 85 Gew.-%, bevorzugt 30 bis 85 Gew.-%,
C) in einer Menge von 0,1 bis 5 Gew.-%, bevorzugt 0.5 bis 4 Gew.-%,
D) in einer Menge von 0 bis 30 Gew.-%, bevorzugt 0 bis 10 Gew.-% und
E) in einer Menge von 0.01 bis 0.5 Gew.-%, bevorzugt 0.01 bis 0.3 Gew.- %,

jeweils bezogen auf die Gesamtmasse der polymerisierbaren Zusammensetzung.

33. Radikalisch polymerisierbare Zusammensetzung nach einem der vorangehenden Aspekte, umfassend

A) in einer Menge von 10 bis 70 Gew.-%, bevorzugt 10 bis 60 Gew.-%,
B) in einer Menge von 20 bis 85 Gew.-%, bevorzugt 30 bis 85 Gew.-%,
C) in einer Menge von 0.1 bis 5 Gew.-%, bevorzugt 0.5 bis 4 Gew.-%,
D) in einer Menge von 0 bis 30 Gew.-%, bevorzugt 0 bis 10 Gew.-%,
E) in einer Menge von 0.01 bis 0.5 Gew.-%, bevorzugt 0.01 bis 0.3 Gew.- %, und
F) in einer Menge von 0.0001 bis 0.5 Gew.-%, bevorzugt 0.0001 bis 0.1 Gew.-%,

jeweils bezogen auf die Gesamtmasse der polymerisierbaren Zusammensetzung.

34. Radikalisch polymerisierbare Zusammensetzung nach einem der vorangehenden Aspekte, die vor der Polymerisation eine Opazität, von kleiner 10% und nach der Polymerisation eine Opazität von größer 50% aufweist.

35. Radikalisch polymerisierbare Zusammensetzung nach einem der vorangehenden Aspekte, die vor der Polymerisation eine Opazität von kleiner 5%, bevorzugt kleiner 3%, und nach der Polymerisation eine Opazität von größer 60%, bevorzugt größer 70%, aufweist.

36. Radikalisch polymerisierbare Zusammensetzung nach einem der vorangehenden Aspekte, die vor der Polymerisation, vorzugsweise unter den angegebenen Messbedingungen, eine Opazität $<10\%$, bevorzugt $<5\%$, besonders bevorzugt $<3\%$, und ganz besonders bevorzugt $<1\%$, aufweist.

37. Radikalisch polymerisierbare Zusammensetzung nach einem der vorangehenden Aspekte, die vor der Polymerisation, vorzugsweise unter den angegebenen Messbedingungen, Transluzenz $\geq 90\%$, bevorzugt $\geq 95\%$, besonders bevorzugt $\geq 97\%$, und ganz besonders bevorzugt $\geq 99\%$, aufweist.

38. Radikalisch polymerisierbare Zusammensetzung nach einem der vorangehenden Aspekte, die nach der Polymerisation, vorzugsweise unter den angegebenen Messbedingungen, eine Opazität $>50\%$, bevorzugt $>60\%$, und besonders bevorzugt $>70\%$, aufweist.

39. Radikalisch polymerisierbare Zusammensetzung nach einem der vorangehenden Aspekte, zur Verwendung in Stereolithographie und/oder 3D-Druck, vorzugsweise zum 3D-Druck von dentalen Formteilen, bevorzugt zum 3D-Druck von dentalen Modellen, Trays, Try-Ins oder Dentures.

40. Radikalisch polymerisierbare Zusammensetzung nach einem der vorangehenden Aspekte, wobei die Zusammensetzung eine dentale radikalisch polymerisierbare Zusammensetzung ist.

41. Radikalisch polymerisierbare Zusammensetzung nach einem der vorangehenden Aspekte zur Verwendung in Stereolithographie und/oder 3D-Druck.

42. Verwendung einer radikalisch polymerisierbaren Zusammensetzung, nach einem der vorangehenden Aspekte, in Stereolithographie und/oder 3D-Druck, vorzugsweise zum 3D-Druck von dentalen Formteilen, bevorzugt zum 3D-Druck von dentalen Modellen, Trays, Try-Ins oder Dentures.

## Patentansprüche

**1.** Radikalisch polymerisierbare Zusammensetzung umfassend

A) 10 bis 80 Gew.-% polymerisierbare (Meth)acryl-Verbindungen der Formel I

$$Y\text{-}O\text{-}[(C_3H_6O)_v\text{-}L^1]_n\text{-}(C_3H_6O)_w\text{-}Z \qquad (I)$$

wobei

$Y = H_2C{=}C(R^1)\text{-}C({=}O)\text{-}$ oder $H_2C{=}C(R^1)\text{-}C({=}O)\text{-}X\text{-}L^2\text{-}$ ,
$R^1$ = H oder $CH_3$,
X = O oder NH,
$L^1$ = divalente organische Linkergruppe,
$L^2$ = divalente organische Linkergruppe,
Z = Y, H oder C1 bis C4-Alkyl,
v = 36 bis 100,
w = 36 bis 200,
v+w = 75 bis 200,
n = 0 bis 3,

B) 15 bis 85 Gew.-% polymerisierbare (Meth)acryl-Verbindungen der Formel II

$$\{H_2C{=}C(R^1)\text{-}C({=}O)\text{-}X\text{-}[CH_2CH(R^2)O]_m\}_qQ \qquad (II)$$

wobei

$R^1$ = H oder $CH_3$,
$R^2$ = H oder $CH_3$,
X = O oder NH,
Q = ein q-valenter organischer Rest,
m = 1 bis 20,
q = 1 bis 4, und

C) 0.01 bis 5 Gew.-% eines Initiators oder Initiatorsystems für die radikalische Polymerisation,

jeweils bezogen auf die Gesamtmasse der polymerisierbaren Zusammensetzung.

**2.** Radikalisch polymerisierbare Zusammensetzung nach Anspruch 1, umfassend

A) in einer Menge von 10 bis 70 Gew.-%, bevorzugt 10 bis 60 Gew.-%,
B) in einer Menge von 20 bis 85 Gew.-%, bevorzugt 30 bis 85 Gew.-% und
C) in einer Menge von 0.1 bis 5 Gew.-%, bevorzugt 0.5 bis 4 Gew.-%,

jeweils bezogen auf die Gesamtmasse der polymerisierbaren Zusammensetzung.

**3.** Radikalisch polymerisierbare Zusammensetzung nach einem der vorangehenden Ansprüche, wobei in der (Meth)acryl-Verbindung der Formel I

X = O,
Z = Y,
n = 0 und
w = 75 bis 180, bevorzugt 75 bis 150, besonders bevorzugt 100 bis 150.

**4.** Radikalisch polymerisierbare Zusammensetzung nach einem der vorangehenden Ansprüche, wobei in der (Meth)acryl-Verbindung der Formel I

X = O,
Z = Y,
n = 1 bis 3,
v = 36 bis 100, bevorzugt 36 bis 90, besonders bevorzugt 36 bis 80,
w = 36 bis 100, bevorzugt 36 bis 90, besonders bevorzugt 36 bis 80 und
v+w = 75 bis 180, bevorzugt 75 bis 150, besonders bevorzugt 100 bis 150.

**5.** Radikalisch polymerisierbare Zusammensetzung nach einem der vorangehenden Ansprüche, wobei in der (Meth)acryl-Verbindung der Formel I $L^1$ eine geradkettige, verzweigte und/oder cyclische Alkylen-, Arylen-, Arylalkylen- oder Alkylarylengruppe ist, die -O-, -OC(=O)-, -C(=O)O-, -NHC(=O)-, -C(=O)NH-, -OC(=O)NH-, oder -NHC(=O)O- enthalten kann, und
$L^2$ eine geradkettige, verzweigte und/oder cyclische Alkylen-, Arylen-, Arylalkylen- oder Alkylarylengruppe ist, die -O-, -OC(=O)-, -C(=O)O-, -NHC(=O)-, -C(=O)NH-, -OC(=O)NH-, oder-NHC(=O)O-enthalten kann, vorzugsweise ist $L^1$ und/oder $L^2$ ausgewählt aus der Gruppe bestehend aus

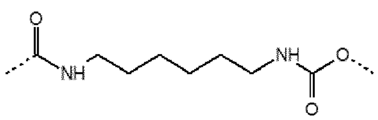 , 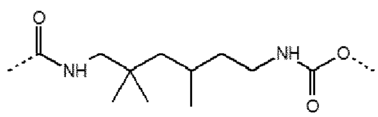 ,

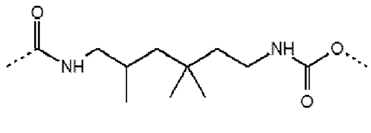, 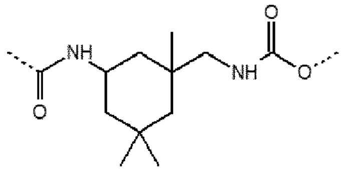,

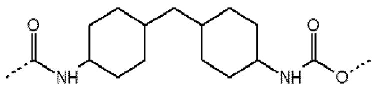, 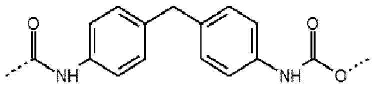,

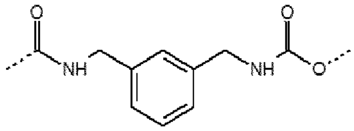 und 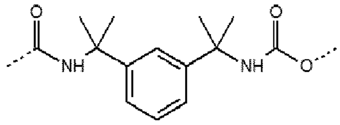,

bevorzugt ausgewählt aus der Gruppe bestehend aus

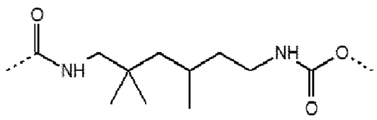, 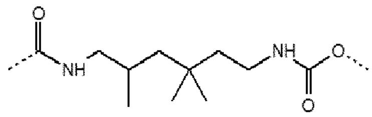

und

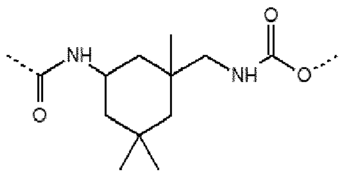,

wobei $L^1$ mit der linken gestrichelten Linie an das äußere Sauerstoffatom der Polypropylengruppe $(C_3H_6O)_v$ gebunden ist und mit der rechten gestrichelten Linie an das äußere Kohlenstoffatom der Polypropylengruppe $(C_3H_6O)_w$ gebunden ist und/oder
wobei $L^2$ mit der linken gestrichelten Linie an X gebunden ist und mit der rechten gestrichelten Linie über ein Sauerstoffatom ( -O- ) an das äußere Kohlenstoffatom einer der Polypropylengruppen $(C_3H_6O)_w$ oder $(C_3H_6O)_v$ gebunden ist.

6. Radikalisch polymerisierbare Zusammensetzung nach einem der vorangehenden Ansprüche, wobei die (Meth)acryl-Verbindung der Formel I ein Molekulargewicht

von größer 5000 g/mol, bevorzugt von größer 6000 g/mol, besonders bevorzugt von größer 7000 g/mol, und/oder
im Bereich von 5000 bis 15000 g/mol, bevorzugt im Bereich von 6000 bis 12000 g/mol, besonders bevorzugt im Bereich von 7000 bis 10000 g/mol,
aufweist.

7. Radikalisch polymerisierbare Zusammensetzung nach einem der vorangehenden Ansprüche, wobei in der (Meth)acryl-Verbindung der Formel II

X = O und/oder
$R^2$ = H.

8. Radikalisch polymerisierbare Zusammensetzung nach einem der vorangehenden Ansprüche, wobei in der

(Meth)acryl-Verbindung der Formel II Q = H, C1 bis C4-Alkyl oder Phenyl, wenn q = 1 oder

Q = $-(CH_2CH_2)-$, $-(CH_2CH(CH_3))-$,

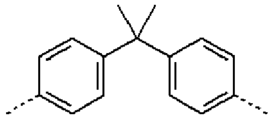

,

wenn q = 2 oder

Q =

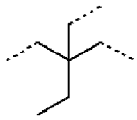

,

wenn q = 3 oder

Q =

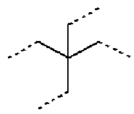

,

wenn q = 4.

**9.** Radikalisch polymerisierbare Zusammensetzung nach einem der vorangehenden Ansprüche, zusätzlich umfassend D) polymerisierbare (Meth)acryl-Verbindungen, die nicht den Formeln I oder II entsprechen.

**10.** Radikalisch polymerisierbare Zusammensetzung nach einem der vorangehenden Ansprüche, die vor der Polymerisation eine Opazität, von kleiner 10% und nach der Polymerisation eine Opazität von größer 50% aufweist.

**11.** Radikalisch polymerisierbare Zusammensetzung nach einem der vorangehenden Ansprüche, die vor der Polymerisation eine Opazität von kleiner 5%, bevorzugt kleiner 3%, und nach der Polymerisation eine Opazität von größer 60%, bevorzugt größer 70%, aufweist.

**12.** Radikalisch polymerisierbare Zusammensetzung nach einem der vorangehenden Ansprüche, wobei die Zusammensetzung eine dentale radikalisch polymerisierbare Zusammensetzung ist.

**13.** Radikalisch polymerisierbare Zusammensetzung nach einem der vorangehenden Ansprüche zur Verwendung in Stereolithographie und/oder 3D-Druck.

**14.** Verwendung einer radikalisch polymerisierbaren Zusammensetzung, nach einem der vorangehenden Ansprüche, in Stereolithographie und/oder 3D-Druck, vorzugsweise zum 3D-Druck von dentalen Formteilen, bevorzugt zum 3D-Druck von dentalen Modellen, Trays, Try-Ins oder Dentures.

Europäisches Patentamt
European Patent Office
Office européen des brevets

# EUROPÄISCHER RECHERCHENBERICHT

Nummer der Anmeldung
EP 23 19 5906

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X,D | WO 2019/175716 A1 (3M INNOVATIVE PROPERTIES CO [US]) 19. September 2019 (2019-09-19)<br>* Seite 1, Zeile 28 - Seite 2, Zeile 9 *<br>* Seite 2, Zeile 33 *<br>* Seite 59; Beispiel 3 *<br>* Seite 61; Beispiel 6 *<br>* Seite 66, Zeile 5 - Seite 67, Zeile 2; Beispiel 24 *<br>* Ansprüche 1,2 *<br>----- | 1-14 | INV.<br>A61C7/08<br>A61K6/16<br>A61K6/887<br>A61K6/62<br>B33Y70/00<br>B33Y80/00 |
| X<br>A | JP H03 212415 A (YUASA BATTERY CO LTD) 18. September 1991 (1991-09-18)<br>* Beispiele 2,4 *<br>----- | 1,2,7, 10-13<br>3-6,8,9, 14 | |
| X<br>A | CN 112 812 265 A (ANQING FEIKAI NEW MAT CO LTD) 18. Mai 2021 (2021-05-18)<br>* Beispiel 4 *<br>* Synthesebeispiel 7 *<br>----- | 1-13<br>14 | |
| | | | RECHERCHIERTE SACHGEBIETE (IPC) |
| | | | A61C<br>A61K<br>B33Y |

Der vorliegende Recherchenbericht wurde für alle Patentansprüche erstellt

1

| Recherchenort | Abschlußdatum der Recherche | Prüfer |
|---|---|---|
| Den Haag | 1. Februar 2024 | Grave, Christian |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
& : Mitglied der gleichen Patentfamilie, übereinstimmendes Dokument

EPO FORM 1503 03.82 (P04C03)

## ANHANG ZUM EUROPÄISCHEN RECHERCHENBERICHT ÜBER DIE EUROPÄISCHE PATENTANMELDUNG NR.

EP 23 19 5906

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

01-02-2024

| Im Recherchenbericht angeführtes Patentdokument | Datum der Veröffentlichung | Mitglied(er) der Patentfamilie | Datum der Veröffentlichung |
|---|---|---|---|
| WO 2019175716 A1 | 19-09-2019 | CN 111868125 A | 30-10-2020 |
| | | EP 3765537 A1 | 20-01-2021 |
| | | JP 2021518449 A | 02-08-2021 |
| | | US 2020332046 A1 | 22-10-2020 |
| | | WO 2019175716 A1 | 19-09-2019 |
| JP H03212415 A | 18-09-1991 | JP H0832755 B2 | 29-03-1996 |
| | | JP H03212415 A | 18-09-1991 |
| CN 112812265 A | 18-05-2021 | KEINE | |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9729901 A1
- WO 2013153183 A2
- WO 2016026915 A1
- WO 2019175716 A1
- WO 2022097667 A1


### In der Beschreibung aufgeführte Nicht-Patentliteratur


- *CHEMICAL ABSTRACTS,* 42405-01-6
- *CHEMICAL ABSTRACTS,* 42404-50-2
- *CHEMICAL ABSTRACTS,* 51243-61-9
- *CHEMICAL ABSTRACTS,* 69790-08-5
- *CHEMICAL ABSTRACTS,* 84434-11-7
- *CHEMICAL ABSTRACTS,* 1834525-17-5
- *CHEMICAL ABSTRACTS,* 25322-69-4
- *CHEMICAL ABSTRACTS,* 25322-68-3
- *CHEMICAL ABSTRACTS,* 822-06-0
- *CHEMICAL ABSTRACTS,* 32052-51-0
- *CHEMICAL ABSTRACTS,* 4098-71-9
- *CHEMICAL ABSTRACTS,* 77-58-7
- *CHEMICAL ABSTRACTS,* 128-37-0
- *CHEMICAL ABSTRACTS,* 868-77-9
- *CHEMICAL ABSTRACTS,* 818-61-1
- *CHEMICAL ABSTRACTS,* 10595-06-9
- *CHEMICAL ABSTRACTS,* 56641-05-5
- *CHEMICAL ABSTRACTS,* 109-16-0
- *CHEMICAL ABSTRACTS,* 25852-47-5
- *CHEMICAL ABSTRACTS,* 41637-38-1
- *CHEMICAL ABSTRACTS,* 28961-43-5
- *CHEMICAL ABSTRACTS,* 51728-26-8
- *CHEMICAL ABSTRACTS,* 7534-94-3
- *CHEMICAL ABSTRACTS,* 5888-33-5
- *CHEMICAL ABSTRACTS,* 2455-24-5
- *CHEMICAL ABSTRACTS,* 5117-12-4
- *CHEMICAL ABSTRACTS,* 15625-89-5
- *CHEMICAL ABSTRACTS,* 4986-89-4
- *CHEMICAL ABSTRACTS,* 6606-59-3
- *CHEMICAL ABSTRACTS,* 43048-08-4
- *CHEMICAL ABSTRACTS,* 72869-86-4
- *CHEMICAL ABSTRACTS,* 63225-53-6
- *CHEMICAL ABSTRACTS,* 66492-51-1
- *CHEMICAL ABSTRACTS,* 29964-84-9